(19) Europäisches Patentamt
European Patent Office
Office européen des brevets

(11) EP 0 574 355 B1

(12) **EUROPEAN PATENT SPECIFICATION**

(45) Date of publication and mention of the grant of the patent:
**13.08.1997 Bulletin 1997/33**

(51) Int Cl.[6]: **C07F 9/09**, A61K 31/66, C07F 9/165, C07F 9/113

(21) Application number: **93810406.4**

(22) Date of filing: **07.06.1993**

(54) **Phosphinyloxy propanaminium inner salt derivatives**

Phosphinyloxy Propanaminium Innersalzderivate

Dérivés de sels internes de phosphinyloxy propanaminium

(84) Designated Contracting States:
**AT BE CH DE DK ES FR GB GR IE IT LI LU NL PT SE**

(30) Priority: **11.06.1992 US 897210**

(43) Date of publication of application:
**15.12.1993 Bulletin 1993/50**

(73) Proprietors:
- **Novartis AG**
  **4058 Basel (CH)**
  Designated Contracting States:
  **BE CH DK ES FR GB GR IE IT LI LU NL PT SE**
- **SANDOZ-PATENT-GMBH**
  **79539 Lörrach (DE)**
  Designated Contracting States:
  **DE**
- **SANDOZ-ERFINDUNGEN Verwaltungsgesellschaft m.b.H.**
  **1235 Wien (AT)**
  Designated Contracting States:
  **AT**

(72) Inventors:
- **Anderson, Robert Charles**
  **Andover, NJ 07821 (US)**
- **Bebernitz, Gregory R.**
  **Warwich, NY 10990 (US)**
- **Fraser, James D.**
  **West Caldwell, NJ 07006 (US)**
- **Hughes, Jeffrey W.**
  **Hopatcong, NJ 07843 (US)**
- **Kapa, Prasad K.**
  **Parsippany, NJ 07054 (US)**
- **Prashad, Mahavir**
  **Hopatcong, NJ 07843 (US)**
- **Smith, Howard C.**
  **Plainsboro, NJ 08536 (US)**
- **Willhauer, Edwin B.**
  **Morristown, NJ 07960 (US)**

(56) References cited:
**EP-A- 0 348 859**
**EP-A- 0 396 082**
**EP-A- 0 402 322**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 574 355 B1

Printed by Jouve, 75001 PARIS (FR)

## Description

The present invention relates to long chain alkyloxy- and aryloxy-substituted phosphinyloxy derivatives of carnitine, in particular to long chain alkoxy- and aryloxy-substituted 3-carboxy-2-phosphinyloxy-1-propanaminium hydroxide, inner salt derivatives.

The invention concerns the compounds of formula (I):

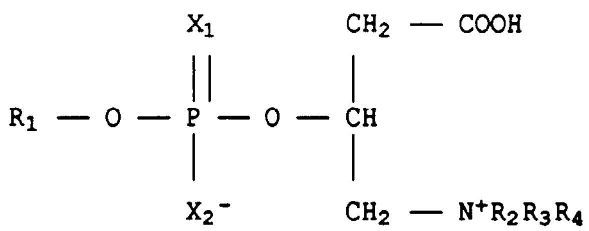

(I)

where

| | |
|---|---|
| $X_1$ and $X_2$ | are independently O or S; |
| R1 is | $R_5$-Y-$R_6$- or $R_7$-Z-$R_8$- where |
| Y | is -O-, -S-, $-CH_2-$, -CH=CH-, -C≡C-, $-N(R_{10})CO-$ or $-CON(R_{10})-$; |
| Z | is -O-, -S- or $-CH_2-$; |
| $R_5$ | is straight or branched chain $(C_{1-17})$alkyl or straight or branched chain ω-trifluoro-$(C_{1-8})$alkyl; |
| $R_6$ | is straight chained $(C_{2-18})$alkylene; and the total number of carbon atoms in $R_5$-Y-$R_6$- is from 7 to 19; |
| $R_7$ | is unsubstituted phenyl, phenoxyphenyl, biphenyl, naphthyl or naphthoxyphenyl; or phenyl, phenoxyphenyl, biphenyl, naphthyl or naphthoxyphenyl mono- or independently di- or independently trisubstituted with halogen, $NO_2$, $NH_2$, CN, $(C_{1-8})$alkyl, $(C_{1-8})$alkoxy, trifluoromethyl, trifluoromethoxy or acetyl; |
| $R_8$ | is straight chained $(C_{3-15})$alkylene, $-(CH_2)_m-N(R_{10})CO-(CH_2)_n-$, $-(CH_2)_m-CON(R_{10})-(CH_2)_n-$ or $-CH_2R_{11}OR_{12}-$ where m and n independently are 1 to 7; |
| $R_{10}$ | is hydrogen, methyl, or ethyl; |
| $R_{11}$ | is straight or branched chain $(C_{1-7})$alkylene; |
| $R_{12}$ | is straight chained $(C_{2-7})$alkylene; and |
| | the total number of carbon atoms in the aryl substituents in $R_7$, and the total number of carbon atoms in $R_8$, not counting significance $R_{10}$, is from 3 to 15; and |
| $R_2$ , $R_3$ and $R_4$ | are each independently straight or branched chain $(C_{1-4})$alkyl; |

in free acid form or in salt, physiologically hydrolysable ester or pro-drug form, hereinafter briefly named "the compounds of the invention".

$X_1$ and $X_2$ preferably are both O or one of $X_1$ and $X_2$ preferably is O; $X_1$ and $X_2$ especially are both 0. $R_1$ preferably is $R_5$-Y-$R_6$-. $R_2$, $R_3$ and $R_4$ preferably are methyl. Y preferably is -O- or $-CH_2-$, especially $-CH_2-$. Z preferably is O. $R_5$ preferably is straight chained, preferably straight chained $(C_{3-8})$alkyl, especially hexyl. $R_6$ preferably is $(C_{3-8})$alkylene, especially heptylene. When Y is -O- or -S-, the total number of carbon atoms in $R_5$-Y-$R_6$- preferably is from 11 to 17; when Y is $-CH_2-$, the total number of carbon atoms in $R_5$-Y-$R_6$- preferably is from 12 to 16; $R_5$-Y-$R_6$- especially is tetradecyl. $R_7$ preferably is optionally substituted phenyl, phenoxyphenyl or naphthyl, it especially is optionally substituted phenyl. When it is substituted phenyl, it preferably is monosubstituted, particularly in the 4 position. $R_8$ preferably is straight chained alkylene, especially $-(CH_2)_{3-6}-$, particularly butylene. $R_{10}$ preferably is hydrogen or methyl. $R_{11}$ and ω-trifluoro-$(C_{1-8})$alkyl preferably are straight chained. The total number of carbon atoms in $R_8$, not counting significance $R_{10}$, preferably is from 5 to 12.

Halogen is fluorine, chlorine, bromine or iodine, it preferably is fluorine or chlorine. $(C_{1-4})$alkyl preferably is methyl. $(C_{1-8})$alkoxy preferably is $(C_{1-6})$alkoxy, it especially is hexyloxy.

Salts, e.g. metal salts such as the sodium or potassium salt and acid addition salts, such as the hydrochloride, can be formed using conventional methods, e.g., for acid addition salts, by reaction with an appropriate acid. Preferred salts are pharmacologically acceptable salts.

Physiologically hydrolysable esters include not only the esters formed with the carboxylic acid group of the carnitine moiety but also orthoesters formed with the phosphate moiety, e.g. the allyl ester. Carboxylic esters can be prepared e.g. by reacting a compound of formula (II) with the desired ester form of the carnitine of formula (III). Phosphate esters

can e.g. be prepared by reacting the product of the reaction of the compound of formula (II) and formula (III) with the desired alcohol before treating the product with an alkali metal perhalate as described hereunder. The invention also includes pro-drug forms of the compounds of formula (I). Such pro-drugs are known and described in the literature, for example in PCT application W091/19721. These esters and pro-drugs include the pivaloyloxymethyl, 4-(2-methoxyphenoxy)-2-methylbutyryloxymethyl, N,N-dimethoxyemethylcarbamoylmethyl, N-(3,6,9-trioxadecyl)-N-methylcarbamoylmethyl, N-(3,6,-dioxaheptyl)-N-methylcarbamoylmethyl, N,N-dipentylcarbamoylmethyl, N,N-dipropylcarbamoylmethyl, N,N-dibutylcarbamoylmethyl, and N-(2-methoxyphenoxyethyl)-N-methylcarbamoylmethyl esters of carnitine.

The compounds of the invention may exist in the form of optically active isomers and can be separated and recovered by conventional techniques. The L-carnitine forms of the compounds are preferred. Compounds in which one of $X_1$ or $X_2$ is a sulfur atom can exist in tautomeric form and in the form of diastereoisomers and can also be separated and recovered by conventional techniques. Similarly, compounds of the invention containing a double bond can exist in the form of geometric isomers, which can be readily separated and recovered by conventional procedures. Such isomeric forms are included in the scope of this invention.

A further subgroup of compounds of the invention is the compounds of formula (Is)

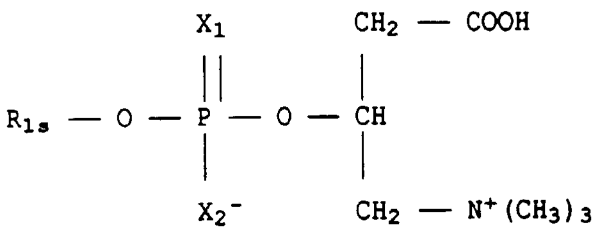

(Is)

where

$X_1$ and $X_2$ are as defined above; and

$R_{1s}$ is $R_5$-$Y_s$-$R_6$- or $R_{7s}$-Z-$R_{8s}$- where

$Y_s$ is -O-, $-CH_2$ -, -CH=CH-, -C≡C-, $-N(CH_3)CO-$ , $-N(CH_2CH_3)CO-$ or $-CON(CH_3)-$ ;

Z, $R_5$ and $R_6$ are as defined above; and the total number of carbon atoms in $R_5$-$Y_s$-$R_6$- is from 7 to 19;

$R_{7s}$ is unsubstituted phenyl, phenoxyphenyl, naphthyl or naphthoxyphenyl; or phenyl, phenoxyphenyl, naphthyl or naphthoxyphenyl mono- or independently di- or independently trisubstituted with fluorine, chlorine, $NO_2$, $NH_2$, CN, $(C_{1-6})$alkyl, $(C_{1-6})$alkoxy, trifluoromethyl, trifluoromethoxy or acetyl;

$R_{8s}$ is straight chained $(C_{3-12})$alkylene, $-(CH_2)_m-N(CH_3)CO-(CH_2)_n-$ , $-(CH_2)_m-CON(CH_3)-(CH_2)_n-$ or $-CH_2R_{11s}OR_{12s}$ where

m and n are as defined above,
$R_{11s}$ is straight or branched chain $(C_{1-4})$alkylene;
$R_{12s}$ is straight chained $(C_{2-5})$alkylene; and

the total number of carbon atoms in the aryl substituents in $R_{7s}$ and the total number of carbon atoms in $R_{8s}$, not counting the methyl group attached to the nitrogen atom, is from 3 to 15;

in free acid form or in salt, or allyl, pivaloyloxymethyl or N,N-diethylcarboxamidylmethyl carboxylic ester or allyl phosphatidic orthoester form.

A further subgroup of compounds of the invention is the compounds of formula (Ip)

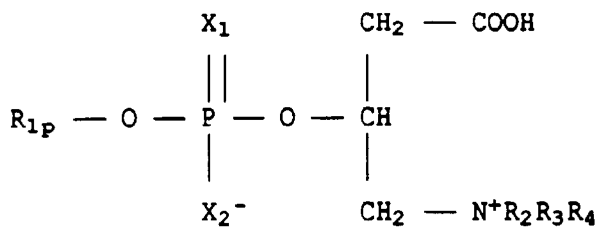

(Ip)

where

| | |
|---|---|
| $X_1$, $X_2$, $R_2$, $R_3$ and $R_4$ | are as defined above and |
| $R_{1p}$ | is $R_{5p}$-$Y_p$-$R_{6p}$- or $R_{7p}$-$Z_p$-$R_{8p}$- where |
| $Y_p$ | is -O-, -S-, -$CH_2$-, -CH=CH- or -C≡C-; |
| $Z_p$ | is -O- or -S-; |
| $R_{5p}$ | is straight or branched chain ($C_{1-17}$)alkyl; |
| $R_{6p}$ | is straight chained ($C_{2-18}$)alkylene; and the total number of carbon atoms in $R_{5p}$-$Y_p$-$R_{6p}$- is from 7 to 19; |
| $R_{7p}$ | is unsubstituted phenyl, biphenyl or naphthyl; or phenyl or naphthyl mono- or independently di- or independently trisubstituted with halogen, $NO_2$, ($C_{1-8}$)alkyl, ($C_{1-8}$)alkoxy, trifluoromethyl, trifluoromethoxy or acetyl; |
| $R_{8p}$ | is straight chained ($C_{3-15}$)alkylene; and the total number of carbon atoms in the substituents in $R_{7p}$ and in $R_{8p}$ is from 3 to 15; |

in free acid form or in pharmaceutically acceptable salt, physiologically hydrolysable ester or pro-drug form.

The compounds of the invention may be prepared by a process comprising

a) appropriately reacting a compound of formula (II)

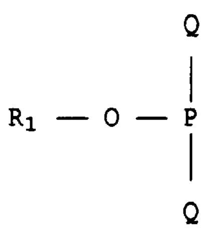

(II)

where $R_1$ is as defined above and Q is chlorine, bromine or iodine, with a compound of formula (III)

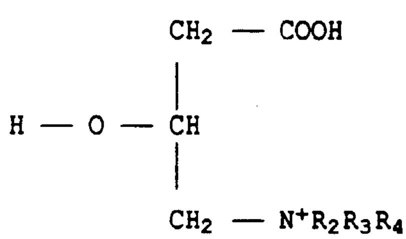

(III)

where $R_2$, $R_3$ and $R_4$ are as defined above, or a hydrolysable ester thereof, and oxidizing or thiolating the product, and hydrolysing or thiolysing the product; or

b) appropriately reacting a compound of formula ($II^i$)

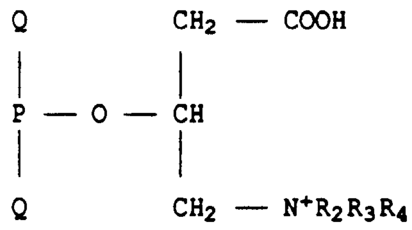

(II$^i$)

where Q, $R_1$, $R_2$ and $R_3$ are as defined above, with a compound of formula (IV)

$$R_1 - OH \qquad (IV)$$

where $R_1$ is as defined above, and oxidizing or thiolating the product, and hydrolysing or thiolysing the product; or c) for the preparation of the compounds of formula I where a phenyl or naphthyl ring in substituent $R_7$ is mono-, di- or trisubstitued with an $NH_2$ group, reducing a corresponding compound mono-, di- or trisubstituted with an $NO_2$ group;

and recovering the resultant compounds in free acid form or in salt, physiologically hydrolysable ester or pro-drug form.

The process of the invention is effected in accordance with conventional procedures.

Process **variant a)** preferably is effected in an inert solvent such as acetonitrile in the presence of a base such as collidine. The oxidation of the product is effected with an appropriate oxidizing agent, preferably with an alkali metal perhalate, e.g. of formula $NaQ'O_4$ wherein Q' is chlorine, bromine or iodine for the preparation of the compounds wherein both $X_1$ and $X_2$ are O. The perhalate preferably is added in water. The temperature preferably is between about 20°C and about 30°C, room temperature is especially preferred. Hydrolysis preferably is effected under alkaline conditions.

For the preparation of the compounds of formula (I) where $X_1$ or $X_2$ is sulfur, the thiolation preferably is effected with sulfur in place of an alkali metal perhalate. For the preparation of the compounds of formula (I) in which both $X_1$ and $X_2$ are sulfur, thiolysis is effected in place of hydrolysis, preferably with hydrogen sulfide.

The compound of formula III conveniently is in the form of the tetrafluoroborate salt $BF_4^-$. In a **variant of process a),** however, no tetrafluoroborate salt is used. The inert solvent preferably is tetrahydrofuran and the base preferably is collidine. The perhalate preferably is added in water. The temperature preferably is as indicated above. It is also preferred that the alkali metal perhalate be added at a reaction temperature maintained between about 0° to 15°C.

Process **variant b)** is effected similarly to process variant a). It preferably is effected in an inert solvent such as acetonitrile and in the presence of a base such as collidine, with the compound of formula (IV) preferably dissolved in e.g. tetrahydrofuran. It also is accompanied by an oxidation or thiolation step with an appropriate oxidizing or thiolating agent, e.g. of formula $NaQ'O_4$, or sulfur, and by hydrolysis or thiolysis, as described above for process variant a). The temperature preferably is about 0°C for the reaction with the compound of formula (IV) and about room temperature for the oxidation or thiolation step. The compound of formula (II$^i$) preferably is in the form of the tetrafluoroborate salt.

Process **variant c)** is effected e.g. by hydrogenation with hydrogen gas over palladium in an inert solvent such as water.

Physiologically hydrolysable esters and pro-drug forms may be obtained e.g. by appropriately esterifying the free acids, e.g., for the preparation of the N,N-diethylcarboxamidylmethyl ester pro-drug form, by reacting the free acid with N,N-diethyl-2-chloroacetamide in e.g. ethyl alcohol. Alternatively, the above process variant a) may be effected using corresponding esterified forms of the starting materials, e.g. of formula III; for example, the pivaloyloxymethyl ester pro-drug forms may be prepared by replacing the compound of formula III by the pivaloyloxymethyl ester thereof in process variant a).

The compounds of the invention may be isolated from the reaction mixture and purified in conventional manner, e.g. by chromatography, e.g. over reverse phase silica gel (C-8) or Amberlite XAD-4 nonionic polymeric adsorbent.

The starting materials may be prepared in conventional manner.

The compounds of formula (II) are prepared e.g. by reacting a compound of formula (IV) with a compound of formula (V)

$$P - Q_3 \qquad (V)$$

where Q is as defined above, preferably in an inert anhydrous solvent under an inert atmosphere.

The compounds of formula ($II^i$) are prepared e.g. by reacting a compound of formula (III) in the form of the tetrafluoroborate salt, with a compound of formula V, preferably in an inert solvent.

The aliphatic alcohols of formula (IV) in which $R_1$ is $R_5$-Y-$R_6$- and Y is oxygen or sulfur may be prepared in accordance with the following reaction scheme:

$$R_5\text{-}M_1 \quad + \quad M_2\text{-}R_6\text{-}M_3 \quad \text{---------}\!> \quad R_5\text{-}Y_1\text{-}R_6\text{-OH}$$

(VI) (VII) ($IV^i$)

where

one or $M_1$ and $M_2$ is Q as defined above and the other is -OH or -SH,
$M_3$ is -OH or protected -OH,
$Y_1$ is -O- or -S-, and
$R_5$ and $R_6$ are as defined above.

They are prepared preferably in an inert solvent in the presence of an alkali metal hydride, deprotecting the compound of formula ($IV^i$) when $M_3$ is protected -OH. Dimethylformamide is the preferred solvent and sodium hydride is the preferred alkali metal hydride. When $M_3$ is a protected hydroxy group, the protecting group can be any conventional hydroxy protecting group, such as dihydropyran. If $M_3$ is protected -OH, the protecting group can be removed by treating the compound with an acid, such as p-toluenesulfonic acid.

Alternatively, the alcohols of formula (IV) in which $R_1$ is $R_5$-O-$R_6$- may be prepared in accordance with the following reaction scheme:

$$R_5'\text{-CH=CH}_2 \quad + \quad \text{HO-}R_6\text{-OH} \quad \xrightarrow[\text{NaOH, NaBH}_4]{\text{HgAc}_2} \quad R_5''\text{-O-}R_6\text{-OH}$$

(VIII) (IX) ($IV^{ii}$)

where

$R_{5'}$ is ($C_{1-15}$)alkyl,
$R_{5''}$ is ($C_{3-17}$)alkyl, and
$R_6$ is as defined above.

They are prepared e.g. by reacting in an inert solvent in the presence of mercuric acetate, and then treating the reactants with aqueous sodium hydroxide and sodium borohydride. Dimethylformamide is the preferred inert solvent.

The amido alcohols of formula (IV) in which $R_1$ is $R_5$-Y-$R_6$- and Y is -N($R_{10}$)CO- may be prepared in accordance with the following reaction scheme:

$$R_5\text{-NH-}R_{10} \quad + \quad \text{Q-CO-}(R_6')\text{-COOR}_2 \quad \text{-----}\!> \quad R_5\text{-N}(R_{10})\text{CO-}R_6'\text{-COOR}_2$$

(X) (XII) (XIII)

$$\xrightarrow{\text{Reduction}} \quad R_5\text{-N}(R_{10})\text{CO-}R_6\text{-OH}$$

($IV^{iii}$)

where

Q, $R_2$, $R_5$, R6, and $R_{10}$ are as defined above and
$R_6'$ is $(C_{1-17})$alkylene.

They are prepared preferably by reacting in an inert solvent, for example methylene chloride, in the presence of a base, such as triethylamine. The intermediate of formula (XIII) obtained is isolated and then reduced preferably in an inert solvent, for example, tertiary butyl alcohol and methanol, with sodium borohydride.

The amido alcohols of formula (IV) in which $R_1$ is $R_7$-Z-$R_8$- and $R_8$ is $-(CH_2)_m-N(R_{10})CO-(CH_2)_n-$ may be prepared similarly in accordance with the following reaction scheme:

$$R_7\text{-Z-}(CH_2)_m\text{-NH-}R_{10} + \text{Q-CO-}(CH_2)_{n-1}\text{-COOR}_2 \text{ ----->}$$

(XIV) (XV)

Reduction

$$R_7\text{-Z-}(CH_2)_m\text{-N}(R_{10})\text{CO-}(CH_2)_{n-1}\text{-COOR}_2 \text{ --------->}$$

(XVI)

$$R_7\text{-Z-}(CH_2)_m\text{-N}(R_{10})\text{CO-}(CH_2)_n\text{-OH}$$

($IV^{iv}$)

where m, n, Q, Z, $R_2$, $R_7$, $R_8$, and $R_{10}$ are as defined above.

They are prepared preferably by reacting a compound of formula (XIV) with a compound of formula (XV) and reducing with sodium borohydride in similar manner as in the preparation of the compounds of formula ($IV^{iii}$) above.

The amido alcohols of formula (IV) in which $R_1$ is $R_5$-Y-$R_6$- and Y is $-CO-N(R_{10})-$ may be prepared in accordance with the following reaction scheme:

$$R_5\text{-CO-Q} + \text{HN}(R_{10})\text{-}(R_6)\text{-OH ----->} R_5\text{-CON}(R_{10})\text{-}R_6\text{-OH}$$

(XVII) (XVIII) ($IV^v$)

where Q, $R_2$, $R_5$, $R_6$, and $R_{10}$ are as defined above.

They are prepared e.g. by adding a compound of formula (XVII) to a compound of formula (XVIII) in an inert solvent, such as methylene chloride, in the presence of a base, such as triethylamine.

The amido alcohols of formula (IV) in which $R_1$ is $R_7$-Z-$R_8$- and $R_8$ is $-(CH_2)_m-CON(R_{10})-(CH_2)_n-$ may be prepared similarly in accordance with the following reaction scheme:

$$R_7\text{-Z-}(CH_2)_m\text{-CO-Q} + \text{HN}(R_{10})\text{-}(CH_2)_n\text{-OH ------>}$$

(XIX) (XX)

$$R_7\text{-Z-}(CH_2)_m\text{-CON}(R_{10})\text{-}(CH_2)_n\text{-OH}$$

($IV^{vi}$)

where m, n, Q, Z, $R_7$, and $R_{10}$ are as defined above, in similar manner as in the preparation of the compounds of formula ($IV^v$) above.

The aromatic alcohols of formula (IV) in which $R_1$ is $R_7$-Z-$R_8$- and Z is oxygen or sulfur may also be prepared in accordance with the following reaction scheme:

$R_7$-ZH + Q-$R_8$-OH ---------> $R_7$-Z-$R_8$-OH

(XXI) (XXII) ($IV^{vii}$)

where Q, Z, $R_7$ and $R_8$ are as defined above.

They are prepared preferably in an inert solvent in the presence of an alkali metal hydride using the same reaction conditions as in the preparation of the compounds of formula (IV') above.

Alternatively, the alcohols of formula ($IV^{vii}$) may be prepared in accordance with the following reaction scheme:

$R_7$-ZH + Q-$R'_8$-$COOR_2$ ---------> $R_7$-Z-$R'_8$-$COOR_2$

(XXI) (XXIII) (XXIV)

Reduction

-----------> $R_7$-Z-$R_8$-OH

($IV^{vii}$)

where

Q, Z, $R_2$, and $R_7$ are as defined above, and

$R'_8$ is straight chained $(C_{3-14})$alkylene, $-(CH_2)_m-N(R_{10})CO-(CH_2)_{n-1}-$, $-R_{11}OR_{12}'-$ , $-(CH_2)_m-CON(R_{10})-(CH_2)_{n-1}-$ or $-CH_2R_{11}OR_{12}-$ , where m, n, $R_{10}$ and $R_{11}$ are as defined above and

$R_{12}'$ is $(C_{2-6})$alkylene.

They are then prepared by reacting a compound of formula (XXI) with an ester of the formula (XXIII) in an inert solvent in the presence of an alkali metal hydride or alkali metal cabonate and then reducing the intermediate of formula (XXIV) with a reducing agent, such as lithium aluminum hydride. The compound of formula (XXIV) can be reduced in situ or isolated first by conventional techniques, for example, chromatography. Reduction is carried out in an inert anhydrous solvent, such as diethyl ether, preferably at reflux temperature.

The compounds of formula (XXI) in which $R_7$ is phenyl or naphthyl substituted by alkoxy of 1 to 8 carbon atoms may e.g. be prepared in accordance with the following reaction scheme:

$R_9$-Q + HO-A-OH ------> $R_9$-O-A-OH

(XXV) (XXVI) (XXVII)

where

A is phenyl or naphthyl and

$R_9$ is $(C_{1-8})$alkyl.

They are prepared preferably by effecting the reaction in an inert solvent in the presence of a halogen scavenging agent, such as potassium carbonate. Dimethylformamide is the preferred inert solvent.

The compounds of formula (II) in which $R_7$ is unsubstituted or substituted phenyl or naphthyl substituted by unsubstituted or substituted phenoxy or naphthoxy may be prepared in accordance with the following reaction scheme:

A'-$M_2'$ + $M_2''$-A''-Z-$R_8$-OH ------> A'-O-A''-Z-$R_8$-OH

(XXVIII) (XXIX) ($IV^{viii}$)

where

A' and A" are each independently phenyl or naphthyl unsubstituted or substituted with halogen, $NO_2$, $NH_2$, CN, $(C_{1-8})$alkyl, $(C_{1-8})$alkoxy, ω-trifluoro-$(C_{1-8})$alkyl, trifluoromethoxy, or acetyl,
one of $M_2$' or $M_2$" is OH and the other is bromo, and
$R_8$ is as defined above.

They are prepared by effecting the reaction preferably in an inert solvent such as pyridine, and in the presence of potassium carbonate and copper oxide.

The alcohols of formula (XXIX) may be prepared in accordance with the following reaction scheme:

$M_2$"-A"-ZH + Q-$R_8$'-$COOR_2$ ------> $M_2$"-A"-Z-$R_8$'-$COOR_2$

(XXX) (XXXI) (XXXII)

Reduction

-----------> $M_2$"-A"-Z-$R_8$-OH

(XXIX)

where $M_2$", A", Z, $R_3$, $R_8$ and $R_8$' are as defined above.

They are prepared preferably by reacting a compound of formula (XXX) with an ester of the formula (XXXI) in an inert solvent, such as dimethylformamide, in the presence of an alkali metal carbonate and then reducing the intermediate of formula (XXXII) with a reducing agent, such as lithium aluminum hydride or DIBAL-H. The compound of formula (XXXII) can be reduced in situ or isolated first by conventional techniques, for example, chromatography. Reduction is carried out in an inert anhydrous solvent, such as diethyl ether or tetrahydrofuran, preferably under an inert atmosphere.

Many of the compounds of formulae (III) to (XXVI) are known and may be prepared by methods described in the literature or may be prepared by analogous methods or as described in the Examples below using known starting materials.

The following Examples illustrate the invention. All temperatures are in degrees Centigrade. **R** or **S** refers to the absolute configuration at the 2 position of the propanaminium moiety; **A** or **B** refers to individual diastereoisomers relative to the configuration at the phosphorus atom.

In the Table: **"ppm"** refers to $^{31}$P-NMR data determined at room temperature using deuterated methanol as solvent; optical rotation values are preceded by **"+"** or **"-"** and unless specified otherwise mean "$[\alpha]_D^{25}$ (c = 1.0, in methanol) "; the indication **"(dec.)"** following a melting point value means "decomposition"; **"ea"** refers to the elemental analysis data at the end of the Table.

## Example 1: (R)-3-Carboxy-N,N,N-trimethyl-2-{[hydroxy(tetradecyloxy)-phosphinyl]oxy}-1-propanaminium hydroxide, inner salt

[Formula I: $X_1$=$X_2$=O; $R_2$=$R_3$=$R_4$= methyl; R isomer; $R_1$ = tetradecyl]

[Process variant a), with tetrafluoroborate; with oxidation and hydrolysis]

To a solution of 16.4 g of the **tetrafluoroborate salt of L-carnitine** (compound of formula III) and 26 g of collidine in 263 ml of acetonitrile is added 29.4 g of **dichlorotetradecylphosphite** (compound of formula II). The reaction mixture is stirred at room temperature for 17 hours and then a solution of 21.1 g of **sodium metaperiodate** in 48.7 ml of water is added. After stirring at room temperature for two hours, the reaction mixture is filtered through Celite and the filtrate is concentrated under vacuum. The residue is flash chromatographed on normal phase silica gel, initially with a 50/30/3 ratio of chloroform/methanol/ concentrated **ammonium hydroxide** and then with a 50/30/10 ratio of the same solvent system to yield a product that is then flash chromatographed on LiChroprep RP-8 silica gel using gradients from water to acetonitrile and to methanol. The **title compound** is isolated as an amorphous solid ($^{31}$P-NMR = 0.324 ppm; m.p. 190° [dec.]; $[\alpha]_D^{25}$ = -10.34° [c=1.0, $CH_3OH$]).

Following the above procedure and using an equivalent amount of **D-carnitine** in place of the L-carnitine, there is obtained the **D-carnitine form (**i.e. the **S enantiomer)** of the title compound ($^{31}$P-NMR = 0.286 ppm; m.p. ~195° [dec.]; $[a]_D^{25}$ = +10.92° [c=0.97, $CH_3OH$]).

The **N,N-diethylcarboxamidylmethyl** [$^{31}$P-NMR = 0.091 ppm; m.p. 60° (softening)] and the **pivaloyloxymethyl**

**ester pro-drug** [$^{31}$P-NMR = 0.081 ppm; m.p. 139° (dec.)] of the title compound may be prepared by reacting the title compound with N,N-diethyl-2-chloroacetamide in ethyl alcohol and triethylamine or, respectively, by replacing the tetrafluoroborate salt of L-carnitine with the tetrafluoroborate salt of the pivaloyloxymethyl ester of L-carnitine in the above reaction.

The starting material is obtained as follows:

Through a solution of 46.8 ml of **$PCl_3$** (compound of formula V) in 200 ml of anhydrous ether and 93.4 ml of anhydrous toluene, dry nitrogen is bubbled for five minutes. Nitrogen is also bubbled through a solution of 20 g of **1-tetradecanol** (compound of formula IV) in 200 ml of anhydrous ether for five minutes. The alcohol solution is added to the $PCl_3$ solution dropwise with stirring over a period of 20 minutes and then stirred for a further two hours. The solvents are removed via distillation to give **dichlorotetradecylphosphite** as a clear colourless liquid.

**Example 2**: **(R)-3-Carboxy-N,N,N-trimethyl-2-{[hydroxy(tetradecyloxy)-phosphinyl]oxy}-1-propanaminium hydroxyde, inner salt**

[Formula I: as for Example 1]

[Process variant a), without tetrafluoroborate; with oxidation and hydrolysis]

The **crude oil** obtained as described hereunder is dissolved in 1.25 l of tetrahydrofuran and 75.5 g of **L-carnitine** is added while maintaining an internal temperature of 25-30°. The suspension formed is stirred for 5 minutes, and a solution of 231 ml of 2,4,6-collidine in 1.25 l of tetrahydrofuran (THF) is added over a period of 40 minutes while maintaining an internal temperature of 25-30°. The resulting white slurry is cooled to 22-23° and stirring is continued for 3 hours. The mixture is cooled to 0-5°, and 750 ml of deionized plant water are added over a period of 10 minutes while maintaining an internal temperature of 5-10°. In three equal 41.7 g portions, a total of 125 g of **sodium periodate** are then added over a period of 30 minutes at 10 minute intervals, while maintaining an internal temperature of 10-15°. The reactants are warmed to 22-23° and stirred an additional 1.5 hour. The mixture is then cooled to 0-5°, and 250 g of sodium thiosulfate dissolved in 500 ml of deionized plant water are added over a period of 30 minutes while maintaining an internal temperature of 5-10°. The reaction mixture is warmed to 22-23° and stirred for 30 minutes. After separating the solids by suction filtration, the filtrate is concentrated under reduced pressure (100 mbar) at 45-50°, until 2.25 l of solvent is collected. The crude mixture obtained is transferred to a 5-1, 4-necked round-bottomed flask equipped with a mechanical stirrer, thermometer, addition funnel, and cooling bath and is diluted with 45 g of **sodium carbonate** dissolved in 350 ml of deionized plant water over a period of 20 minutes while maintaining an internal temperature of 22-23°. After addition is complete, 2.0 l of ethyl acetate are added, and the two-phase mixture is stirred for 30 minutes while maintaining an internal temperature of 22-23°. The layers are separated, and the organic phase is discarded. The aqueous layer is cooled to 0-5° and 120 ml of concentrated hydrochloric acid are added over a period of 30 minutes while maintaining an internal temperature of 5-10°. The resulting solution is warmed to 22-23° to yield 2.3 l of an aqueous solution of crude **title compound.**

To the crude product are added 400 g of reverse phase silica gel (C-8), and the resulting slurry is stirred for 45 minutes. The gel obtained is collected by filtration over a 3-liter glass fritted funnel using suction. This treatment of the filtrate is repeated twice with 400 g and then with a 200 g portion of reverse phase silica gel. The gel fractions are mixed together and eluted with HPLC grade water using suction until the conductance of the filtrate reaches <100 µS/cm. This is followed by 7.5 l of absolute ethanol. The first 0.82 l of solvent, collected after switching to elution with ethanol, is discarded. The next 5.9 l of solvent collected is concentrated under reduced pressure (25-30 mmHg) at 40-45° until 5.5 l of solvent is collected. The resulting thick slurry is transferred, with the aid of 400 ml of absolute ethanol to a 5-1, 4-necked, round-bottomed flask equipped with a mechanical stirrer, thermometer, addition funnel and heating mantle and warmed to 55-60°. One liter of solution is obtained, to which 1.8 l of THF is added over a period of 30 minutes while maintaining an internal temperature of 50-55°. After cooling to 22-23° over 2 hours and stirring for an additional 1 hour, the solids are collected by filtration under suction and washed with three 100 ml portions of THF. The solids are transferred to a 2-1, 4-necked, round-bottomed flask, and 475 ml of 95 % ethanol are added. The suspension obtained is warmed to 50-55°, and the resulting solution is filtered under suction into a 5-1, 4-necked, round bottomed flask equipped with a heating mantle, thermometer, mechanical stirrer and addition funnel. This solution is warmed to 50-55°, and 1.25 l of THF are added over a period of 30 minutes while maintaining an internal temperature of 50-55°. The mixture is cooled to 22-23° over 2.0 hours and stirred for an additional 1.0 hour. The solids formed are collected by filtration under suction and washed with a total of 300 ml of THF in three equal portions of 100 ml each followed by 150 ml of acetone. The solids are then dried at 45-50° at 25-30 mmHg for about 16 hours until the weight is constant to obtain the pure **title compound.**

Alternatively, the above purification can also be carried out using Amberlite XAD-4 nonionic polymeric adsorbent in place of the expensive reverse phase silica gel (C-8) by treating 1.5 kg of Amberlite XAD-4 with the crude product

solution above in the same manner as described for the reverse phase silica gel (C-8). By eluting with water followed by 7.5 l of absolute ethanol, 5.9 l of solution containing the desired product is obtained. Evaporation of 5.5 l of ethanol from the resulting solution results in a slurry, which is dissolved in an additional 400 ml of ethanol by heating to 55-60° to yield 1 l of solution. The pure **title compound** is precipitated from the solution by adding 1.8 l of THF and then dried ($^{31}$P-NMR = 0.324 ppm; m.p. 190° [dec.]; $[\alpha]_D^{25}$ = -10.34° [c=1.0, $CH_3OH$]).

The starting material is obtained as follows:

A 5-1, 4-necked round-bottomed flask, equipped with a mechanical stirrer, thermometer, addition funnel, drying tube and cooling bath is charged with 153 ml of **phosphorus trichloride** and 625 ml of heptane; and 125 g of **1-tetradecanol** dissolved in 625 ml of heptane are added over a period of 50-55 minutes while maintaining an internal temperature of 23-25°. This reaction mixture is stirred for 45 minutes and then concentrated at 40-45° under a reduced pressure of 25-30 mmHg until 1.2 l of solvent is removed. **Dichlorotetradecylphosphite** is obtained as a crude oil.

**Example 3**: **(R)-3-Carboxy-N,N,N-trimethyl-2-{[hydroxy(tetradecyloxy)-phosphinothioyl]oxy}-1-propanaminium hydroxyde, inner salt**

[Formula I: $X_1$=S; $X_2$=O; $R_2$=$R_3$=$R_4$= methyl; R isomer; $R_1$ = tetradecyl]

[Process variant a); with thiolation and hydrolysis]

To a solution of 7.8 g of the **tetrafluoroborate salt of L-carnitine** and 11.2 g of collidine in 100 ml of acetonitrile is added 14.7 g of **dichlorotetradecylphosphite** in 50 ml of acetonitrile. The reaction mixture is stirred at room temperature for 1.5 hours and then the solvents are removed under vacuum. The resulting residue is dissolved in a mixture of 400 ml of pyridine and 20 ml of water that had been saturated with nitrogen gas and is stirred under a nitrogen atmosphere at room temperature for 30 minutes. Next, 7.4 g of **sulfur** is added and the mixture is stirred for a further 18 hours. The reaction mixture is filtered through Celite, and the filtrate is concentrated under vacuum. The resulting residue is flash chromatographed on silica gel, initially with a 50/30/3 ratio of methylene chloride/methanol/concentrated **ammonium hydroxide** and then with a 50/30/5 ratio of the same solvent system to give a **diastereomeric mixture** of the desired material. Additional low pressure chromatography using a 300/400/60/72 ratio of hexane/isopropanol/ water/ isopropylamine yields crude isomer A and isomer B. The individual isomers are further purified by flash chromatography in silica gel eluting initially with a 50/30/3 ratio of methylene chloride/methanol/concentrated ammonium hydroxide and then with a 50/30/6 ratio of the same solvent system. Further purification is achieved by flash chromatography on LiChroprep RP-8 silica gel, eluting initially with water and then with ethanol, to give the pure individual diastereomers as amorphous solids:

**isomer A**: $^{31}$P-NMR = 57.5 ppm; $[\alpha]_D^{25}$ = -21.3° [c=1.0, $CH_3OH$]);

**isomer B**: $^{31}$P-NMR = 58.2 ppm; $[\alpha]_D^{25}$ = -14.8° [c=1.0, $CH_3OH$]).

**Example 4**: **(R)-3-Carboxy-N,N,N-trimethyl-2-{[mercapto(tetradecyloxy)-phosphinothioyl]oxy}-1-propanaminium hydroxyde, inner salt**

[Formula I: $X_1$=X2=S; $R_2$=$R_3$=$R_4$= methyl; R isomer; $R_1$ = tetradecyl]

[Process variant a); with thiolation and thiolysis]

To a solution of 1.2 g of the **tetrafluoroborate salt of L-carnitine** and 1.7 g of collidine in 10 ml of acetonitrile is added 5.7 g of **dichlorotetradecylphosphite** in 15 ml of acetonitrile. The reaction mixture is stirred at room temperature for 1 hour and then **hydrogen sulfide** gas is bubbled through the mixture for five minutes. The reaction mixture is stirred at room temperature an additional fifteen minutes, and then the solvents are removed under vacuum. The resulting residue is dissolved in 50 ml of pyridine and 1 ml of water and 1.1 g of **sulfur** is added, and the mixture is stirred at room temperature for 17 hours. The reaction mixture is filtered through Celite and the filtrate is concentrated under vacuum. The resulting residue is flash chromatographed on silica gel, initially with a 50/30/5 ratio of methylene chloride/methanol/concentrated ammonium hydroxide and then with a 50/30/5 ratio of the same solvent system. Subsequent flash chromatography on LiChroprep RP-8 silica gel eluting initially with water and then with methanol gives the **title compound** as an amorphous solid ($^{31}$P-NMR = 115.57 ppm; m.p. 178° [dec.]).

**Example 5:** **(R)-3-Carboxy-N,N,N-trimethyl-2-{[hydroxy((2-propenyl)-oxytetradecyloxy)phosphinyl]oxy}-1-propanaminium hydroxide, inner salt)**

[Formula I: $X_1$=X2=O; $R_2$=$R_3$=$R_4$= methyl; R isomer; $R_1$ = tetradecyl; pro-drug form as allyl orthoester with phosphate moiety]

[Process variant a); with oxidation and hydrolysis]

To a solution of 2.0 g of the **tetrafluoroborate salt of L-carnitine** in 32 ml of acetonitrile and 3.2 ml of collidine, 3.8 g of **dichlorotetradecylphosphite** are added and the reaction mixture is stirred at room temperature for 24 hours. To this is then added 2.7 ml of **allyl alcohol** and the solution is stirred at room temperature for 2 hours. A solution of 2.6 g of **$NaIO_4$** in 10.0 ml of water is added and the reaction mixture is stirred for 2 hours. The mixture is then filtered through Celite, which is subsequently washed with methanol and concentrated under vacuum to give a white, oily solid. This is flash chromatographed, initially with a 50/10/0.5 ratio of chloroform/methanol/concentrated ammonium hydroxide and subsequently with a 50/30/3 ratio. The ammonium hydroxide salt is then flash chromatographed through a reverse phase plug (LiChroprep RP-8 silica gel) to give the **title compound** as an off-white solid [$^{31}$P-NMR = -1.280 ppm, -1.315 ppm; m.p. -100° (softening)].

**Example 6: (R)-N,N,N-trimethyl-4-oxo-4-[(2-propenyl)oxy)-2- [[hydroxy(tetradecyloxy)phosphinyl]oxyl]-1-butanaminium hydroxide, inner salt**

[Formula I: $X_1$=X2=O; $R_2$=$R_3$=$R_4$= methyl; R isomer; $R_1$ = tetradecyl; allyl ester with the carboxylic group]

[Process variant a); with oxidation and hydrolysis]

Proceeding analogously to Example 1 but replacing the tetrafluoroborate salt of L-carnithine by the **tetrafluoroborate salt of the allyl ester of L-carnithine,** the **title compound** is obtained (amorphous solid) [$^{31}$P-NMR = 0.108 ppm].

**Example 7**: **(R)-3-Carboxy-N,N,N-trimethyl-2-[[hydroxy(N-ethyl-N-heptylhexamidyl-6-oxy)phosphinyl]oxy]-1-propanaminium hydroxyde, inner salt**

[Formula I: $X_1$=X2=O; $R_2$=$R_3$=$R_4$= methyl; R isomer; $R_1$ = $R_5$-Y-$R_6$- where $R_5$ = heptyl, $R_6$ = pentamethylene and Y = -N($C_2H_5$)CO-]

[Process variant b); with oxidation and hydrolysis]

A solution of 1.0 g of **N-heptyl-N-ethyl(6-hydroxy)hexanamide** [compound of formula (IV)] in 4.0 ml of tetrahydrofuran is syringe-added to the **crude product** [compound of formula ($II^i$)] obtained as described hereunder, and the reaction mixture is stirred at ice-water temperature for 2 hours. To this mixture is added a solution of 0.83 g of **$NaIO_4$** in 3 ml of water, following which it is stirred at room temperature for 18 hours and then concentrated under vacuum. The residue obtained is flash chromatographed, initially with a 50/30/3 ratio of $CH_2Cl_2$/MeOH/**$NH_4OH$** and then, after filtering off the non-polar impurities, with a 50/30/10 ratio. The isolated ammonium hydroxide inner salt is then flash chromatographed through a reverse phase plug (LiChroprep RP-8 silica gel) to yield the **title compound** as a light yellow solid [$^{31}$P-NMR = -0.046 ppm; m.p. 175° (dec.)]

The starting material of formula ($II^i$) is obtained as follows: A solution containing 1.45 g of the **tetrafluoroborate salt of L-carnitine** and 2.05 ml of collidine in 20 ml of acetonitrile is cooled to -40°, and 0.34 ml of **phosphorous trichloride** is syringe-added. The reaction is stirred at -40° for 10 minutes and then at ice-water temperature for 2 hours, and the crude reaction mixture is used without further purification.

The starting material of formula (IV) is obtained as follows: To a solution of 5.0 g of **1-aminoheptane** in 150 ml of $CH_2Cl_2$ is added 10.9 ml of **$Et_3N$** followed by 2.79 ml of **acetyl chloride.** The reaction mixture is stirred at room temperature for 18 hours and then concentrated under vacuum. The residue obtained is partitioned between ethyl acetate and an aqueous 2N HCl solution; and after separating, the organic layer is washed with aqueous saturated $NaHCO_3$, then brine, and dried over $Na_2SO_4$. After filtration and concentration, **N-heptylacetamide** is obtained as a clear liquid.

A mixture of a solution of 5.5 g of N-heptylacetamide in 150 ml of diethyl ether and 3.99 g of **lithium aluminum hydride** are stirred at room temperature for 18 hours. The reaction mixture is then worked up with $H_2O$/NaOH by the Fieser method, following which the organic layer is dried over MgSO4, filtered, and concentrated under vacuum to give **N-heptyl-N-ethylamine.**

To a solution of 3.2 g of N-heptyl-N-ethylamine in 96 ml of $CH_2Cl_2$ are added 5.7 ml of **$Et_3N$** followed by 3.63 ml of **hexanedioic acid monochloride monomethyl ester.** This mixture is stirred at room temperature for 18 hours; and after concentrating under vacuum, the residue obtained is partitioned between ethyl acetate and an aqueous 2N HCl solution. The organic layer is separated and washed with aqueous saturated NaHCO3, then brine, and dried over MgSO4. Following filtration and concentration under vacuum, the residue is flash chromatographed on normal phase silica gel using a 3/1 ratio of hexane and ethyl acetate to yield **N-heptyl-N-ethyladipamic acid, methyl ester.**

A mixture of a solution of 3.1 g of N-heptyl-N-ethyladipamic acid, methyl ester in 40 ml of tert-butanol and 800 mg of sodium borohydride is heated to reflux, following which 7.8 ml of methanol is added dropwise. The reaction mixture is stirred at reflux for 18 hours and then partitioned between water and $CH_2Cl_2$. After separation, the organic layer is dried over $MgSO_4$, filtered, and concentrated under vacuum. The residue obtained is flash chromatographed on normal phase silica gel with a 4/1 ratio of hexane and ethyl acetate to yield **N-heptyl-N-ethyl-(6-hydroxy)hexanamide.**

**Example 8: (R)-3-Carboxy-N,N,N-trimethyl-2-[[hydroxy[8-(2-aminophenoxy)-octyloxy]phosphinyl]oxy]-1-propanaminium hydroxide, inner salt**

[Formula I: $X_1$=$X_2$=O; $R_2$=$R_3$=$R_4$= methyl; R isomer; $R_1$ = $R_7$-Z-$R_8$- where $R_7$ = 2-aminophenyl, Z = -O- and $R_8$ = octamethylene]

[Process variant c) (reduction)]

A solution of 490 mg of **compound of Example 68** in 5 ml of water and 490 mg of 10% Pd on carbon are flushed in a suitable flask with **$H_2$ gas** and stirred under an $H_2$-containing balloon for 2 days. The reaction mixture is added to a column of LiChroprep RP-8 silica gel and eluted sequentially with water, acetonitrile, and methanol. The product eluted in the methanol is evaporated to yield the **title compound** [$^{31}$P-NMR = 0.381 ppm; $[\alpha]_D^{25}$ = -10.07° (c=1, $CH_3OH$)].

The following compounds of the invention (formula I) are obtained in analogous manner from corresponding starting materials:

| Ex. No. | $X_1$ | $X_2$ | $R_1$ | $R_2$ | $R_3$ | $R_4$ | Isomer | Process variant | Analog.to Ex.No. | Character. data |
|---|---|---|---|---|---|---|---|---|---|---|
| 9 | O | O | tridecyl | $CH_3$ | $CH_3$ | $CH_3$ | R | a) | 1 | 0.094 ppm; 190° (dec.) |
| 10 | O | O | pentadecyl | $CH_3$ | $CH_3$ | $CH_3$ | R | a) | 1 | 0.098 ppm; 190° (dec.) |
| 11 | O | O | 7-(Z)-tetradecenyl | $CH_3$ | $CH_3$ | $CH_3$ | R | a) | 1 | 0.078 ppm |
| 12 | O | O | 11-(Z)-tetradecenyl | $CH_3$ | $CH_3$ | $CH_3$ | R | a) | 1 | 0.115 ppm |
| 13 | O | O | 7-tetradecynyl | $CH_3$ | $CH_3$ | $CH_3$ | R | a) | 1 | 0.059 ppm |
| 14 | O | O | 4-nonyloxybutyl | $CH_3$ | $CH_3$ | $CH_3$ | R | a) | 1 [1] | 0.050 ppm; 190° (dec.) |
| 15 | O | O | 12-methyloxydodecyl | $CH_3$ | $CH_3$ | $CH_3$ | R | a) | 14 [2] | 0.077 ppm; 190° (dec.) |
| 16 | O | O | 6-heptyloxyhexyl | $CH_3$ | $CH_3$ | $CH_3$ | R | a) | 14 | 0.074 ppm; 190° (dec.) |
| 17 | O | O | 8-pentyloxyoctyl | $CH_3$ | $CH_3$ | $CH_3$ | R | a) | 14 | 0.105 ppm; 190° (dec.) |
| 18 | O | O | 10-propyloxydecyl | $CH_3$ | $CH_3$ | $CH_3$ | R | a) | 14 | 0.064 ppm; 190° (dec.) |
| 19 | O | O | 2-undecyloxyethyl | $CH_3$ | $CH_3$ | $CH_3$ | R | a) | 14 | 0.002 ppm; 190° (dec.) |
| 20 | O | O | 9-(3,3-dimethylbutyloxy)nonyl | $CH_3$ | $CH_3$ | $CH_3$ | R | a) | 14 | 0.066 ppm; 195° (dec.) |
| 21 | O | O | 5-octyloxypentyl | $CH_3$ | $CH_3$ | $CH_3$ | R | a) | 14 | 0.294 ppm; 197° (dec.) |
| 22 | O | O | 5-(3-decyloxy)pentyl | $CH_3$ | $CH_3$ | $CH_3$ | R | a) | 14 | 0.040 ppm; 185° (dec.) |
| 23 | O | O | 9-(4,4,4-trifluorobutyloxy)nonyl | $CH_3$ | $CH_3$ | $CH_3$ | R | a) | 14 | 0.052 ppm; 185° (dec.) |
| 24 | O | O | 6-(2-nonyloxy)pentyl | $CH_3$ | $CH_3$ | $CH_3$ | R | a) | 1 [3] | 0.001 ppm; 195° (dec.) |
| 25 | O | O | 6-(2-octyloxy)hexyl | $CH_3$ | $CH_3$ | $CH_3$ | R | a) | 24 | 0.288 ppm; 197° (dec.) |
| 26 | O | O | 4-(2-decyloxy)butyl | $CH_3$ | $CH_3$ | $CH_3$ | R | a) | 24 | 0.012 ppm; 197° (dec.) |
| 27 | O | O | 8-[2-(2-methyl)hexyloxy]octyl | $CH_3$ | $CH_3$ | $CH_3$ | R | a) | 24 | 0.240 ppm; 185° (dec.) |
| 28 | O | O | 9-[2-(2-methyl)pentyloxy]nonyl | $CH_3$ | $CH_3$ | $CH_3$ | R | a) | 24 | 0.238 ppm; 198° (dec.) |
| 29 | O | O | 7-[2-(2-methyl)heptyloxy]heptyl | $CH_3$ | $CH_3$ | $CH_3$ | R | a) | 24 | 0.058 ppm; 197° (dec.) |
| 30 | O | O | 5-[2-(2-methyl)nonyloxy]pentyl | $CH_3$ | $CH_3$ | $CH_3$ | R | a) | 24 | 0.278 ppm; 197° (dec.) |
| 31 | O | O | 8-(3-methylpentyloxy)octyl | $CH_3$ | $CH_3$ | $CH_3$ | R | a) | 1 [4] | 0.284 ppm; 197° (dec.) |
| 32 | O | O | 8-[4-(trifluoromethoxy)phenoxy]octyl | $CH_3$ | $CH_3$ | $CH_3$ | R | a) | 1 [5] | -1.156 ppm; 174° (dec.) |
| 33 | O | O | 7-phenoxyheptyl | $CH_3$ | $CH_3$ | $CH_3$ | R | a) | 32 | -0.812 ppm; 160° (dec.) |
| 34 | O | O | 7-(4-chlorophenoxy)heptyl | $CH_3$ | $CH_3$ | $CH_3$ | R | a) | 32 | 0.036 ppm; 165° (dec.) |

| Ex. No. | $X_1$ | $X_2$ | $R_1$ | $R_2$ | $R_3$ | $R_4$ | Isomer | Process variant | Analog.to Ex.No. | Character. data |
|---|---|---|---|---|---|---|---|---|---|---|
| 35 | O | O | 6-(4-chlorophenoxy)hexyl | $CH_3$ | $CH_3$ | $CH_3$ | R | a) | 32 | 0.073 ppm; 165° (dec.) |
| 36 | O | O | 8-(4-chlorophenoxy)octyl | $CH_3$ | $CH_3$ | $CH_3$ | R | a) | 32 | 0.326 ppm; 165° (dec.) |
| 37 | O | O | 9-(4-chlorophenoxy)nonyl | $CH_3$ | $CH_3$ | $CH_3$ | R | a) | 32 | 0.106 ppm |
| 38 | O | O | 8-(2-chlorophenoxy)octyl | $CH_3$ | $CH_3$ | $CH_3$ | R | a) | 32 | 0.332 ppm; 173° (dec.) |
| 39 | O | O | 8-(3-chlorophenoxy)octyl | $CH_3$ | $CH_3$ | $CH_3$ | R | a) | 32 | -1.166 ppm; 180° (dec.) |
| 40 | O | O | 7-(1-naphthyloxy)heptyl | $CH_3$ | $CH_3$ | $CH_3$ | R | a) | 32 | 0.162ppm; 165-70°(dec.) |
| 41 | O | O | 7-(2-naphthyloxy)heptyl | $CH_3$ | $CH_3$ | $CH_3$ | R | a) | 32 | 0.142ppm; 170-75°(dec.) |
| 42 | O | O | 7-(3,5-di-trifluoromethylphenoxy)heptyl | $CH_3$ | $CH_3$ | $CH_3$ | R | a) | 32 | 0.153 ppm; 185° (dec.) |
| 43 | O | O | 8-(4-tert-butylphenoxy)octyl | $CH_3$ | $CH_3$ | $CH_3$ | R | a) | 32 | 0.337 ppm; 182° (dec.) |
| 44 | O | O | 8-(4-phenylphenoxy)octyl | $CH_3$ | $CH_3$ | $CH_3$ | R | a) | 32 | 0.138 ppm; 190° (dec.) |
| 45 | O | O | 8-(4-acetyl-3-methylphenoxy)octyl | $CH_3$ | $CH_3$ | $CH_3$ | R | a) | 32 | -1.214 ppm; 172° (dec.) |
| 46 | O | O | 4-[3-(2-naphthoxy)phenoxy]butyl | $CH_3$ | $CH_3$ | $CH_3$ | R | a) | 109 | 0.060 ppm; 175° (dec.) |
| 47 | O | O | 8-[(4-chlorophenyl)thio]octyl | $CH_3$ | $CH_3$ | $CH_3$ | R | a) | 32 | 0.269 ppm; 185° (dec.) |
| 48 | O | O | 10-(4-chlorophenoxy)decyl | $CH_3$ | $CH_3$ | $CH_3$ | R | a) | 32 | 0.109 ppm; 190° (dec.) |
| 49 | O | O | 8-(3,5-dimethoxyphenoxy)octyl | $CH_3$ | $CH_3$ | $CH_3$ | R | a) | 32 | 0.268 ppm; 159° (dec.) |
| 50 | O | O | 8-(2,3,4-trichlorophenoxy)octyl | $CH_3$ | $CH_3$ | $CH_3$ | R | a) | 32 | 0.264 ppm; 168° (dec.) |
| 51 | O | O | 8-(2,5-dinitrophenoxy)octyl | $CH_3$ | $CH_3$ | $CH_3$ | R | a) | 32 | 0.131 ppm; 158° (dec.) |
| 52 | O | O | 8-(2,3-dimethylphenoxy)octyl | $CH_3$ | $CH_3$ | $CH_3$ | R | a) | 32 | 0.269 ppm;-8.08°;186-88°(dec) |
| 53 | O | O | 8-(3,4-dimethylphenoxy)octyl | $CH_3$ | $CH_3$ | $CH_3$ | R | a) | 32 | 0.321ppm;-11.51°;190-92°(dec) |
| 54 | O | O | 8-(3-fluoro-4-nitrophenoxy)octyl | $CH_3$ | $CH_3$ | $CH_3$ | R | a) | 32 | 0.309 ppm; -7.20° |
| 55 | O | O | 8-(2,4-dimethylphenoxy)octyl | $CH_3$ | $CH_3$ | $CH_3$ | R | a) | 32 | 0.323 ppm; -9.69° |
| 56 | O | O | 8-(4-nitrophenoxy)octyl | $CH_3$ | $CH_3$ | $CH_3$ | R | a) | 32 | -9.53°; 192-94° (dec.) |
| 57 | O | O | 8-(3-nitrophenoxy)octyl | $CH_3$ | $CH_3$ | $CH_3$ | R | a) | 32 | 0.097 ppm;174-76°(dec.) |
| 58 | O | O | 8-(2,4-dinitrophenoxy)octyl | $CH_3$ | $CH_3$ | $CH_3$ | R | a) | 32 | 0.119 ppm; -9.14° |
| 59 | O | O | 8-(2,4-dichlorophenoxy)octyl | $CH_3$ | $CH_3$ | $CH_3$ | R | a) | 32 | 0.169 ppm; -6.84° |
| 60 | O | O | 8-(3-trifluoromethoxyphenoxy)octyl | $CH_3$ | $CH_3$ | $CH_3$ | R | a) | 32 | 0.339 ppm; -7.96° |
| 61 | O | O | 8-(2-trifluoromethylphenoxy)octyl | $CH_3$ | $CH_3$ | $CH_3$ | R | a) | 32 | 0.463ppm;-8.50°;175-77°(dec) |

| Ex. No. | $X_1$ | $X_2$ | $R_1$ | $R_2$ | $R_3$ | $R_4$ | Isomer | Process variant | Analog.to Ex.No. | Character. data |
|---|---|---|---|---|---|---|---|---|---|---|
| 62 | O | O | 8-(4-methoxyphenoxy)octyl | $CH_3$ | $CH_3$ | $CH_3$ | R | a) | 32 | 0.333 ppm; -9.37° |
| 63 | O | O | 4-(6-propoxy-2-naphthoxy)butyl | $CH_3$ | $CH_3$ | $CH_3$ | R | a) | 32 | 0.295ppm; 163-65°(dec.) |
| 64 | O | O | 8-(2,3-dichlorophenoxy)octyl | $CH_3$ | $CH_3$ | $CH_3$ | R | a) | 32 | 0.338 ppm; -8.25° |
| 65 | O | O | 8-(2,5-dichlorophenoxy)octyl | $CH_3$ | $CH_3$ | $CH_3$ | R | a) | 32 | 0.325 ppm; -7.69° |
| 66 | O | O | 8-(4-methylphenoxy)octyl | $CH_3$ | $CH_3$ | $CH_3$ | R | a) | 32 | 0.125 ppm; -10.04° |
| 67 | O | O | 8-(4-trifluoromethylphenoxy)octyl | $CH_3$ | $CH_3$ | $CH_3$ | R | a) | 32 | 0.126ppm;-7.67°;186°(dec.) |
| 68 | O | O | 8-(2-nitrophenoxy)octyl | $CH_3$ | $CH_3$ | $CH_3$ | R | a) | 32 | 0.339 ppm; -8.56° |
| 69 | O | O | 9-(4-trifluoromethoxyphenoxy)nonyl | $CH_3$ | $CH_3$ | $CH_3$ | R | a) | 32 | 0.465ppm;-8.85°;193-5°(dec) |
| 70 | O | O | 8-(2,6-dichlorophenoxy)octyl | $CH_3$ | $CH_3$ | $CH_3$ | R | a) | 32 | 0.340 ppm; -8.61° |
| 71 | O | O | 3-[3-pentyloxyphenoxy]propyl | $CH_3$ | $CH_3$ | $CH_3$ | R | a) | 1 [6) | -0.745 ppm |
| 72 | O | O | 3-[3-hexyloxyphenoxy]propyl | $CH_3$ | $CH_3$ | $CH_3$ | R | a) | 71 | 0.182 ppm;150-95°(dec.) |
| 73 | O | O | 5-[3-butyloxyphenoxy]pentyl | $CH_3$ | $CH_3$ | $CH_3$ | R | a) | 71 | -0.591 ppm |
| 74 | O | O | 5-[3-pentyloxyphenoxy]pentyl | $CH_3$ | $CH_3$ | $CH_3$ | R | a) | 71 | -0.737 ppm |
| 75 | O | O | 4-[3-pentyloxyphenoxy]butyl | $CH_3$ | $CH_3$ | $CH_3$ | R | a) | 1 [7) | 0.296 ppm; 190° (dec.) |
| 76 | O | O | 4-[3-hexyloxyphenoxy]butyl | $CH_3$ | $CH_3$ | $CH_3$ | R | a) | 75 | 0.281 ppm; 192° (dec.) |
| 77 | O | O | 4-[3-butyloxyphenoxy]butyl | $CH_3$ | $CH_3$ | $CH_3$ | R | a) | 75 | -0.752 ppm |
| 78 | O | O | 4-[5-methyl-3-pentyloxyphenoxy]butyl | $CH_3$ | $CH_3$ | $CH_3$ | R | a) | 75 | 0.247 ppm |
| 79 | O | O | 4-[5-methoxy-3-pentyloxyphenoxy]butyl | $CH_3$ | $CH_3$ | $CH_3$ | R | a) | 75 | 0.381 ppm;179-81°(dec.) |
| 80 | O | O | 4-[2,4-dichloro-5-pentyloxyphenoxy]butyl | $CH_3$ | $CH_3$ | $CH_3$ | R | a) | 75 | 0.250ppm; 173-75°(dec.) |
| 81 | O | O | 4-[2-methyl-3-pentyloxyphenoxy]butyl | $CH_3$ | $CH_3$ | $CH_3$ | R | a) | 75 | 0.293 ppm; -9.24° |
| 82 | O | O | 4-[3-cyano-5-pentyloxyphenoxy]butyl | $CH_3$ | $CH_3$ | $CH_3$ | R | a) | 75 | -0.866 ppm; -6.96° |
| 83 | O | O | 4-[4-butyloxyphenoxy]butyl | $CH_3$ | $CH_3$ | $CH_3$ | R | a) | 75 | 3.299 ppm |
| 84 | O | O | 4-[7-propoxy-2-naphthalenyloxy]butyl | $CH_3$ | $CH_3$ | $CH_3$ | R | a) | 75 | 0.278 ppm:; 175° (dec.) |
| 85 | O | O | 4-[5-butoxy-1-naphthalenyloxy]butyl | $CH_3$ | $CH_3$ | $CH_3$ | R | a) | 75 | 0.046 ppm:; 180° (dec.) |
| 86a | S | O | 4-[3-pentyloxyphenoxy]butyl | $CH_3$ | $CH_3$ | $CH_3$ | R (A) | a) | 3 | 57.8 ppm; -17.7° |
| 86b | S | O | 4-[3-pentyloxyphenoxy]butyl | $CH_3$ | $CH_3$ | $CH_3$ | R (B) | a) | 3 | 58.3 ppm; -12.1° |
| 87a | S | O | 4-[5-methyl-3-pentyloxyphenoxy]butyl | $CH_3$ | $CH_3$ | $CH_3$ | R (A) | a) | 3 | 57.9ppm; -20.1°;155-59° |
| 87b | S | O | 4-[5-methyl-3-pentyloxyphenoxy]butyl | $CH_3$ | $CH_3$ | $CH_3$ | R (B) | a) | 3 | 58.3ppm; -13.0°;153-68° |

| Ex. No. | $X_1$ | $X_2$ | $R_1$ | $R_2$ | $R_3$ | $R_4$ | Isomer | Process variant | Analog.to Ex.No. | Character. data |
|---|---|---|---|---|---|---|---|---|---|---|
| 88a | S | O | 8-[4-trifluoromethoxyphenoxy]octyl | $CH_3$ | $CH_3$ | $CH_3$ | R (A) | a) | 3 | 57.8ppm; -18.8°;157-61° |
| 88b | S | O | 8-[4-trifluoromethoxyphenoxy]octyl | $CH_3$ | $CH_3$ | $CH_3$ | R (B) | a) | 3 | 58.2ppm; -12.9°;148-60° |
| 89a | S | O | 4-[3-hexyloxyphenoxy]butyl | $CH_3$ | $CH_3$ | $CH_3$ | R (A) | a) | 3 | 57.7 ppm; -19.8° |
| 89b | S | O | 4-[3-hexyloxyphenoxy]butyl | $CH_3$ | $CH_3$ | $CH_3$ | R (B) | a) | 3 | 58.3 ppm; -12.8° |
| 90 | S | S | 4-[3-pentyloxyphenoxy]butyl | $CH_3$ | $CH_3$ | $CH_3$ | R | a) | 4 | 115.6ppm; -17.5°;85-105° |
| 91 | O | O | 4-[4-phenoxyphenoxy]butyl | $CH_3$ | $CH_3$ | $CH_3$ | R | a) | 1 [8] | 0.074 ppm; -8.84° |
| 92 | O | O | 4-[3-phenoxyphenoxy]butyl | $CH_3$ | $CH_3$ | $CH_3$ | R | a) | 91 | 0.170 ppm; -7.74° |
| 93 | O | O | 4-[(3-(4-trifluoromethoxy)phenoxy)-phenoxy]butyl | $CH_3$ | $CH_3$ | $CH_3$ | R | a) | 91 | 0.286 ppm; -7.5° |
| 94 | O | O | 4-[(4-(4-trifluoromethoxy)phenoxy)-phenoxy]butyl | $CH_3$ | $CH_3$ | $CH_3$ | R | a) | 91 | 0.078 ppm; -7.54° |
| 95 | O | O | 4-[4-(1-naphthoxy)phenoxy]butyl | $CH_3$ | $CH_3$ | $CH_3$ | R | a) | 91 | 0.082 ppm; 170° (dec.) |
| 96 | O | O | 4-[3-(2-naphthoxy)phenoxy]butyl | $CH_3$ | $CH_3$ | $CH_3$ | R | a) | 91 | 0.060 ppm; 175° (dec.) |
| 97 | O | O | N-methyl-N-heptyl-hexanamid-6-yl | $CH_3$ | $CH_3$ | $CH_3$ | R | a) | 7 | 0.008 ppm |
| 98 | O | O | N-methyl-N-hexyl-heptanamid-7-yl | $CH_3$ | $CH_3$ | $CH_3$ | R | a) | 7 | 0.092 ppm; 155° (dec.) |
| 99 | O | O | N-methyl-N-[2-(4-chlorophenoxy)ethyl]-hexanamid-6-yl | $CH_3$ | $CH_3$ | $CH_3$ | R | a) | 7 | 0.038 ppm; 180° (dec.) |
| 100 | O | O | N-methyl-N-hexanoyl-7-aminoheptyl | $CH_3$ | $CH_3$ | $CH_3$ | R | a) | 7 [9] | 0.115 ppm; 180° (dec.) |
| 101 | O | O | N-methyl-N-heptanoyl-6-aminohexyl | $CH_3$ | $CH_3$ | $CH_3$ | R | a) | 100 | 0.308 ppm; 175° (dec.) |
| 102 | O | O | N-methyl-N-heptanoyl-5-aminopentyl | $CH_3$ | $CH_3$ | $CH_3$ | R | a) | 100 | 0.086 ppm; 175° (dec.) |
| 103 | O | O | N-methyl-N-[2-(4-chlorophenoxy)acetyl]-6-aminohexyl | $CH_3$ | $CH_3$ | $CH_3$ | R | a) | 100 | 0.125 ppm; 175° (dec.) |
| 104 | O | O | 5-[3-pentyloxyphenyl]pentyl | $CH_3$ | $CH_3$ | $CH_3$ | R | a) | 1 [10] | 0.283 ppm; 188° |
| 105 | O | O | 5-[3-hexylphenyl]pentyl | $CH_3$ | $CH_3$ | $CH_3$ | R | a) | 1 [11] | 0.353ppm; >179°(dec.) |
| 106 | O | O | 5-[4-(4-chlorophenoxy)-2-butyloxy]pentyl | $CH_3$ | $CH_3$ | $CH_3$ | R | a) | 1 [12] | 0.177 ppm; 185°(dec.) |
| 107 | O | O | 8-(4-aminophenoxy)octyl | $CH_3$ | $CH_3$ | $CH_3$ | R | c) | 8 [13] | 0.383 ppm |
| 108 | O | O | 4-[(3-(4-trifluoromethoxy)phenoxy)-phenoxy]butyl | $CH_3$ | $CH_3$ | $CH_3$ | R | a) | 1 [14] | 0.286 ppm; -7.5° |

| Ex. No. | $X_1$ | $X_2$ | $R_1$ | $R_2$ | $R_3$ | $R_4$ | Isomer | Process variant | Analog.to Ex.No. | Character. data |
|---|---|---|---|---|---|---|---|---|---|---|
| 109 | O | O | 4-[(4-(4-trifluoromethoxy)phenoxy)-phenoxy]butyl | $CH_3$ | $CH_3$ | $CH_3$ | R | a) | 1 [15] | 0.078 ppm; -7.54° |

[1] The starting material **4-nonyloxybutanol** (compound of formula II) is used in place of tetradecanol and is obtained by reacting 1,4-butanediol in dimethylformamide with sodium hydride and then adding bromononane to the product of the reaction;

[2] As for [1], using iodomethane in place of bromononane and 1,12-dodecanediol in place of 1,4-butanediol;

[3] The starting material **5-(2-nonyloxy)pentanol** is obtained by reacting 1,5-pentanediol in dimethylformamide with mercuric acetate and 1-nonene and reducing the resultant mixture with $NaBH_4$ solution in aqueous NaOH;

4) The starting material **8-(3-methylpentyloxy)octanol** is obtained by reacting 8-bromo-1-octanol in anhydrous diethyl ether with p-toluenesulfonic acid in dihydropyran; the resultant protected bromo-alcohol is reacted with 3-methylpentanol in dimethylformamide in the presence of sodium hydride, and the resultant product is reacted in methanol with p-toluenesulfonic acid, the methanol removed under reduced pressure, the residue partitioned between ethyl acetate and water, the organic layer washed with aqueous $NaHCO_3$ and brine, dried, filtered and concentrated;

5) The starting material **8-[4-(trifluoromethoxy)phenoxy]-1-octanol** is obtained by reacting 4-(trifluoromethoxy)-phenol with 8-bromo-1-octanol in anhydrous dimethylformamide at 80° in the presence of $K_2CO_3$;

6) The starting material **3-[3-(pentyloxy)phenoxy]propanol** is obtained by reacting 1-bromopentane with resorcinol in dimethylformamide in the presence of potassium carbonate and reacting the resultant 3-pentyloxyphenol with NaH in dimethylformamide in the presence of 3-bromopropanol;

7) The starting material **4-[3-(pentyloxy)phenoxy]butanol** is obtained by reacting 3-pentyloxyphenol and ethyl 4-bromobutyrate with NaH in dimethylformamide and reducing the resultant ethyl 4-(3-pentyloxyphenoxy) butyrate with $LiAlH_4$ in anhydrous ether;

8) The starting material **4-[(4-phenoxy)phenoxy]butanol** is obtained by reacting 4-(4-phenoxy)phenol with ethyl 4-bromobutyrate in anhydrous dimethylformamide in the presence of $K_2CO_3$ and reducing the resultant ethyl 4-[(4-phenoxy)phenoxy]butyrate with $LiAlH_4$ in anhydrous ethyl ether under a nitrogen atmosphere;

9) The intermediate **N-(7-hydroxyheptyl)-N-methylhexanamide** (compound of formula IV) is obtained by reacting 7-bromoheptanol in dioxane with 40 % aqueous methylamine and reacting the resultant HBr salt of 7-(N-methylamino)heptanol in $CH_2Cl_2$ with hexanoyl chloride and triethylamine;

10) The intermediate **3-(5-hydroxypentyl)phenol, n-pentyl ether** (compound of formula IV) used in place of 1-tetradecanol is obtained by reacting 3-bromophenol in dimethylformamide with 1-bromopentane in the presence of $K_2CO_3$, reacting the resultant 3-bromophenol, n-pentyl ether in tetrahydrofuran with t-butyllithium and then $Li_2CuCl_3$, reacting the resultant mixture with 1-bromo-5-benzyloxypentane, and after chromatography over silicagel (1:1 hexane:ether), hydrogenating the resultant crude (3-benzyloxypentyl)phenol, n-pentyl ether in ethanol over 5 % palladium on carbon;

11) The intermediate **5-(3-hexylphenyl)pentanol** (compound of formula IV) used in place of 1-tetradecanol is obtained by reacting the Wittig salt prepared from 1-bromopentane and triphenylphosphine in tetrahydrofuran with n-butyllithium in hexane and then 3-bromobenzaldehyde, reacting the resultant 3-bromo-(1-hexenyl)benzene (after chromatography over silicagel eluting with 3:2 hexane:ether) in tetrahydrofuran with t-butyllithium in hexane and $Li_2CuCl_3$ in tetrahydrofuran and then adding 1-bromo-5-benzyloxypentane, purifying the resultant 3-(5-benzyloxypentyl)-(1-hexenyl)benzene by HPLC chromatography, and hydrogenating the pure compound in methanol with 5 % palladium on carbon;

12) The intermediate **1-methyl-3-(p-chlorophenoxy)propyl-5-hydroxypentylether** (compound of formula IV) used in place of 1-tetradecanol is obtained by reacting p-chlorophenol in dimethylformamide with 1-bromo-3-butene in the presence of $K_2CO_3$, reacting the resultant p-chlorophenyl-3-butenyl ether with 1,5-pentanediol in the presence of mercuric acetate in dimethylformamide, adding aqueous NaOH solution and reacting the mixture with $NaBH_4$;

13) Using as starting material the **compound of Example 56** in place of the compound of Example 68;

14) The intermediate **4-[3-[4-(trifluoromethoxy)phenoxy]phenoxy]butan-1-ol** (compound of formula IV) used in place of 1-tetradecanol is obtained by reacting 3-bromophenol with ethyl 4-bromobutyrate in dimethylformamide in the presence of $K_2CO_3$, reacting the resultant ethyl 4-(3-bromophenoxy)butyrate in tetrahydrofuran with DIBAL-H in toluene and reacting the resultant 4-(3-bromophenoxy)butan-1-ol with 4-(trifluoromethoxy)phenol and CuO in the presence of $K_2CO_3$ and pyridine;

15) The intermediate **4-[4-[4-(trifluoromethoxy)phenoxy]phenoxy]butan-1-ol** (compound of formula IV) used in place of 1-tetradecanol is obtained by reacting p-hydroquinone with ethyl 4-bromobutyrate in dimethylformamide in the presence of $K_2CO_3$, reacting the resultant ethyl 4-(4-hydroxyphenoxy)butyrate in ether with $LiAlH_4$, and reacting the resultant 4-(4-hydroxyphenoxy)butan-1-ol with 4-(trifluoromethoxy)bromobenzene and CuO in the presence of $K_2CO_3$ and pyridine.

The compounds of formula (I) in free acid form or in pharmaceutically acceptable salt or pharmaceutically acceptable and physiologically hydrolysable ester pro-drug form, hereinafter briefly named **"the agents of the invention",** exhibit pharmacological activity in animals. In particular, they have hypoglycemic activity and are thus indicated for use in the treatment of diabetes, as indicated by standard tests, e.g. by the acute and chronic hypoglycemic screen test in male Sprague-Dawley rats given from about 1 mg/kg to about 100 mg/kg of agent. The rats, 2 to 3 months of age, weighing 250 g in the acute screen test and 200 g to 220 g in the chronic screen test, are kept in a room at a controlled ambient temperature of 22°C and a 12/12 hour light/dark cycle for at least seven days in the acute screen test and seven days before and during testing the chronic screen test.

In the **acute** screen test, Purina rat chow and water are available ad libitum. Following an 18 hours fast, rats (5/group) are given test compounds orally by gavage in 0.5 % carboxymethylcellulose (CMC) with 0.2 % Tween $80^R$. The animals receive 1.0 ml/100 g body weight. Three hours after administration, the rats are anesthetized with $CO_2$ and blood is collected via cardiac puncture. Sera are collected and used for glucose and β-hydroxybutyrate determination. Glucose is measured by the glucose oxidase method (YSI Model 27, Yellow Spring, Ohio) and β-hydroxybutyrate is assayed with a β-hydroxybutyrate dehydrogenase-linked enzyme assay (Sigma Kit 310-uv - St. Louis, Mo.). The $ED_{50}$ value is the amount of agent required to produce a 50 % inhibition of maximum reduction in β-hydroxybutyrate level.

In the **chronic** screen test, the rats are fed a high fat diet ad libitum. At fed state, 40 mg of streptozotocin/kg of body weight are injected via the tail vein. 5 days later, those rats are considered to be diabetic which have fed blood glucose of greater than 200 mg/dl and, following an overnight fast, when given an oral glucose tolerance test, have blood glucose of 40 mg/dl to 80 mg/dl three hours after the test. Four days later, animals are used in the screen, if fed blood glucose levels are greater than 180 mg/dl. Blood glucose is determined with a YSI Glucose Analyzer. The chronic screen test is carried out as follows:

On day 1, food is removed from rats at 8:00 a.m.; and after an initial blood glucose reading is taken via the tip of the tail, vehicle (control) or agent and vehicle (9 rats/treatment) is administered orally. Six hours later blood glucose level is measured; and immediately thereafter the rats are refed. The same rats are given either vehicle or drug once a day for 11 consecutive days. Blood glucose is determined at 0 hour and after a 6-hour fast, post dosing on days 4, 8 and 11. The $ED_{50}$ value calculated on day 11 is the amount of agent required to produce 50 % of maximal efficacy in ability of an agent to normalize blood glucose levels. Efficacy of 100 % represents restoration of blood glucose levels to that of a normal rat.

The antidiabetically effective dosage of the agents of the invention employed for the alleviation of the above condition will vary depending on the particular compound employed, the mode of administration and severity of the condition being treated. However, in general, satisfactory results are obtained when the agents are administered at a daily dosage of from about 1 mg to about 100 mg per kilogram of animal body weight, optionally given in divided doses two to four times a day, or in sustained release form. For the larger mammals, for example primates such as humans, the total daily dosage is from about 1 mg to about 1000 mg per day. Unit dosage forms comprise from about 1 mg, preferably about 5 mg to about 250 mg of the agent in intimate admixture with a solid or liquid pharmaceutically acceptable carrier or diluent.

The agents of the invention may be administered in a manner similar to known standards for the above uses. The suitable daily dosage for a particular agent will depend on a number of factors, such as its relative potency of activity. It has for example been determined that the compound of Example 1 has an $ED_{50}$ of 4.2 mg/kg in the acute and 45 mg/kg/day in the chronic hypoglycemia test, and the compound of Example 76 has an $ED_{50}$ of 1.6 mg/kg in the acute and an $ED_{70}$ of 37 mg/kg/day in the chronic hypoglycemia test. An indicated daily dose for these two agents is from about 1 mg to about 1000 mg, preferably about 20 mg to about 250 mg p.o. for the larger primates, such as humans.

For the above use, the agents of the invention may be administered orally or parenterally as such or admixed with conventional pharmaceutical carriers. They may be administered orally in such forms as tablets, dispersible powders, granules, capsules, syrups and elixirs, and parenterally as solutions or emulsions. These pharmaceutical preparations may contain up to about 90 % of the active ingredient in combination with the carrier or adjuvant.

Capsules containing the ingredients indicated below may be prepared by conventional techniques.

Example of a capsule:

| Ingredient | Weight (mg) |
|---|---|
| Compound of Example 1 or 76 | 25 |
| Microcrystalline cellulose | 65.6 |
| Colloidal silicon dioxide | 0.16 |

(continued)

| Ingredient | Weight (mg) |
|---|---|
| Mannitol | 65.6 |
| Hydrogenated castor oil | 6.64 |
| | total 163 |

Preferred are:

- the compound of Examples 1 and 2; and
- the compound of Example 76, i.e. (R)-3-carboxy-N,N,N-trimethyl-2-{[hydroxy[4-(3-hexyloxyphenoxy)butyloxy] phosphinyl]oxy}-1-propanaminium hydroxide, inner salt,

especially the compound of Example 1.

The invention thus also concerns a pharmaceutical composition comprising an agent of the invention together with at least one pharmaceutically acceptable carrier or diluent. It further concerns a process for the preparation of a pharmaceutical composition which comprises mixing an agent of the invention together with at least one pharmaceutically acceptable carrier or diluent.

## Claims

**1.** A compound of formula (I)

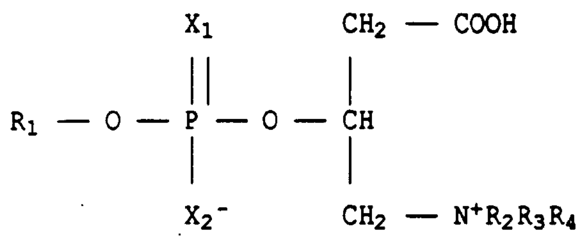

(I)

where

$X_1$ and $X_2$ are independently O or S;

$R_1$ is $R_5$-Y-$R_6$- or $R_7$-Z-$R_8$- where

Y is -O-, -S-, $-CH_2-$, -CH=CH-, -C≡C-, $-N(R_{10})CO-$ or $-CON(R_{10})-$;

Z is -O-, -S- or $-CH_2-$;

$R_5$ is straight or branched chain $(C_{1-17})$alkyl or straight or branched chain ω-trifluoro-$(C_{1-8})$alkyl;

$R_6$ is straight chained $(C_{2-18})$alkylene; and the total number of carbon atoms in $R_5$-Y-$R_6$- is from 7 to 19;

$R_7$ is unsubstituted phenyl, phenoxyphenyl, biphenyl, naphthyl or naphthoxyphenyl; or phenyl, phenoxyphenyl, biphenyl, naphthyl or naphthoxyphenyl mono- or independently di- or independently trisubstituted with halogen, $NO_2$, $NH_2$, CN, $(C_{1-8})$alkyl, $(C_{1-8})$alkoxy, trifluoromethyl, trifluoromethoxy or acetyl;

$R_8$ is straight chained $(C_{3-15})$alkylene, $-(CH_2)_m-N(R_{10})CO-(CH_2)_n-$, $-(CH_2)_m-CON(R_{10})-(CH_2)_n-$ or $-CH_2R_{11}OR_{12}-$ where m and n independently are 1 to 7;

$R_{10}$ is hydrogen, methyl, or ethyl;

$R_{11}$ is straight or branched chain $(C_{1-7})$alkylene;

$R_{12}$ is straight chained $(C_{2-7})$alkylene; and the total number of carbon atoms in the aryl substituents in $R_7$, and the total number of carbon atoms in $R_8$, not counting significance $R_{10}$, is from 3 to 15; and

$R_2$, $R_3$ and $R_4$ are each independently straight or branched chain $(C_{1-4})$alkyl;

in free acid form or in salt, physiologically hydrolysable ester or pro-drug form.

**2.** A compound according to claim 1 of formula (Is)

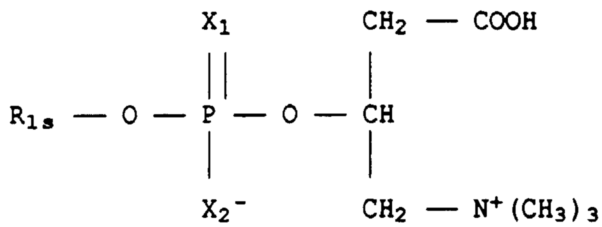

(Is)

where

$X_1$ and $X_2$ are as defined in claim 1; and

$R_{1s}$ is $R_5$-$Y_s$-$R_6$- or $R_{7s}$-Z-$R_{8s}$- where

$Y_s$ is -O-, $-CH_2-$, -CH=CH-, -C≡C-, $-N(CH_3)CO-$ , $-N(CH_2CH_3)CO-$ or $-CON(CH_3)-$ ;

Z, $R_5$ and $R_6$ are as defined in claim 1; and the total number of carbon atoms in $R_5$-$Y_s$-$R_6$- is from 7 to 19;

$R_{7s}$ is unsubstituted phenyl, phenoxyphenyl, naphthyl or naphthoxyphenyl; or phenyl, phenoxyphenyl, naphthyl or naphthoxyphenyl mono- or independently di- or independently trisubstituted with fluorine, chlorine, $NO_2$, $NH_2$, CN, $(C_{1-6})$alkyl, $(C_{1-6})$alkoxy, trifluoromethyl, trifluoromethoxy or acetyl;

$R_{8s}$ is straight chained $(C_{3-12})$alkylene, $-(CH_2)_m-N(CH_3)CO-(CH_2)_n-$, $-(CH_2)_m-CON(CH_3)-(CH_2)_n-$ or $-CH_2R_{11s}OR_{12s}$ where

m and n are as defined in claim 1,
$R_{11s}$ is straight or branched chain $(C_{1-4})$alkylene;
$R_{12s}$ is straight chained $(C_{2-5})$alkylene; and

the total number of carbon atoms in the aryl substituents in $R_{7s}$ and the total number of carbon atoms in $R_{8s}$, not counting the methyl group attached to the nitrogen atom, is from 3 to 15;

in free acid form or in salt, or allyl, pivaloyloxymethyl or N,N-diethylcarboxamidylmethyl carboxylic ester or allyl phosphatidic orthoester form.

**3.** A compound according to claim 1 of formula (Ip)

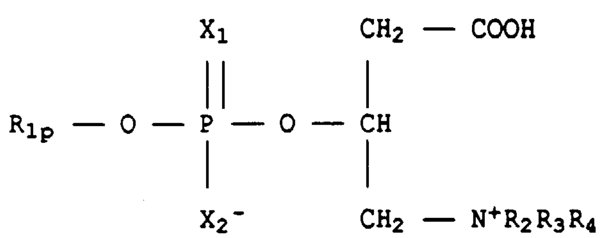

(Ip)

where

$X_1$, $X_2$, $R_2$, $R_3$ and $R_4$ are as defined in claim 1 and

$R_{1p}$ is $R_{5p}$-$Y_p$-$R_{6p}$- or $R_{7p}$-$Z_p$-$R_{8p}$- where

- $Y_p$ is -O-, -S-, -$CH_2$-, -CH=CH- or -C≡C-;
- $Z_p$ is -O- or -S-;
- $R_{5p}$ is straight or branched chain ($C_{1-17}$)alkyl;
- R6p is straight chained ($C_{2-18}$)alkylene; and the total number of carbon atoms in $R_{5p}$-$Y_p$-$R_{6p}$- is from 7 to 19;
- $R_{7p}$ is unsubstituted phenyl, biphenyl or naphthyl; or phenyl or naphthyl mono- or independently di- or independently trisubstituted with halogen, $NO_2$, ($C_{1-8}$)alkyl, ($C_{1-8}$)alkoxy, trifluoromethyl, trifluoromethoxy or acetyl;
- $R_{8p}$ is straight chained ($C_{3-15}$)alkylene; and the total number of carbon atoms in the substituents in $R_{7p}$ and in $R_{8p}$ is from 3 to 15;

in free acid form or in pharmaceutically acceptable salt, physiologically hydrolysable ester or pro-drug form.

**4.** The compound according to claim 1 of formula (I) where $R_2$, $R_3$ and $R_4$ are methyl and

either $X_1$, $X_2$, $R_1$ and the isomer form respectively are :

S, O, tetradecyl and R(AB); or
S, O, tetradecyl and R(A); or
S, O, tetradecyl and R(B); or
S, S, tetradecyl and R; or
S, O, 4-[3-pentyloxyphenoxy]butyl and R(A); or
S, O, 4-[3-pentyloxyphenoxy]butyl and R(B); or
S, O, 4-[5-methyl-3-pentyloxyphenoxy]butyl and R(A); or
S, O, 4-[5-methyl-3-pentyloxyphenoxy]butyl and R(B); or
S, O, 8-[4-trifluoromethoxyphenoxy]octyl and R(A); or
S, O, 8-[4-trifluoromethoxyphenoxy]octyl and R(B); or
S, O, 4-[3-hexyloxyphenoxy]butyl and R(A); or
S, O, 4-[3-hexyloxyphenoxy]butyl and R(B); or
S, S, 4-[3-pentyloxyphenoxy]butyl and R;

or $X_1$ and $X_2$ are O and $R_1$ and the isomer form respectively are: tetradecyl and S;
or $X_1$ and $X_2$ are O, the isomer form is R, and $R_1$ is :

tetradecyl (in N,N-diethylcarboxamidylmethyl carboxylic ester pro-drug form); tetradecyl (in pivaloyloxymethyl carboxylic ester pro-drug form); tetradecyl (in allyl phosphatidic orthoester pro-drug form); tetradecyl (in allyl carboxylic ester form); N-ethyl-N-heptyl-hexanamid-6-yl;
8-(2-aminophenoxy)octyl; tridecyl; pentadecyl; 7-(Z)-tetradecenyl;
11-(Z)-tetradecenyl; 7-tetradecynyl; 4-nonyloxybutyl; 12-methyloxydodecyl;
6-heptyloxyhexyl; 8-pentyloxyoctyl; 10-propyloxydecyl; 2-undecyloxyethyl;
9-(3,3-dimethylbutyloxy)nonyl; 5-octyloxypentyl; 5-(3-decyloxy)pentyl;
9-(4,4,4-trifluorobutyloxy)nonyl; 6-(2-nonyloxy)pentyl;
6-(2-octyloxy)hexyl; 4-(2-decyloxy)butyl; 8-[2-(2-methyl)hexyloxy]octyl;
9-[2-(2-methyl)pentyloxy]nonyl; 7-[2-(2-methyl)heptyloxy]heptyl;
5-[2-(2-methyl)nonyloxy]pentyl; 8-(3-methylpentyloxy)octyl;
8-[4-(trifluoromethoxy)phenoxy]octyl; 7-phenoxyheptyl;
7-(4-chlorophenoxy)heptyl; 6-(4-chlorophenoxy)hexyl;
8-(4-chlorophenoxy)octyl; 9-(4-chlorophenoxy)nonyl;
8-(2-chlorophenoxy)octyl; 8-(3-chlorophenoxy)octyl;
7-(1-naphthyloxy)heptyl; 7-(2-naphthyloxy)heptyl;
7-(3,5-di-trifluoromethylphenoxy)heptyl; 8-(4-tert-butylphenoxy)octyl;
8-(4-phenylphenoxy)octyl; 8-(4-acetyl-3-methylphenoxy)octyl;
4-[3-(2-naphthoxy)phenoxy]butyl; 8-[(4-chlorophenyl)thio]octyl;
10-(4-chlorophenoxy)decyl; 8-(3,5-dimethoxyphenoxy)octyl;
8-(2,3,4-trichlorophenoxy)octyl; 8-(2,5-dinitrophenoxy)octyl;

8-(2,3-dimethylphenoxy)octyl; 8-(3,4-dimethylphenoxy)octyl; 8-(3-fluoro-4-nitrophenoxy)octyl; 8-(2,4-dimethylphenoxy)octyl; 8-(4-nitrophenoxy)octyl; 8-(3-nitrophenoxy)octyl; 8-(2,4-dinitrophenoxy)octyl; 8-(2,4-dichlorophenoxy)octyl; 8-(3-trifluoromethoxyphenoxy)octyl; 8-(2-trifluoromethylphenoxy)octyl; 8-(4-methoxyphenoxy)octyl; 4-(6-propoxy-2-naphthoxy)butyl; 8-(2,3-dichlorophenoxy)octyl; 8-(2,5-dichlorophenoxy)octyl; 8-(4-methylphenoxy)octyl; 8-(4-trifluoromethylphenoxy)octyl; 8-(2-nitrophenoxy)octyl; 9-(4-trifluoromethoxyphenoxy)nonyl; 8-(2,6-dichlorophenoxy)octyl; 3-[3-pentyloxyphenoxy]propyl; 3-[3-hexyloxyphenoxy]propyl; 5-[3-butyloxyphenoxy]pentyl; 5-[3-pentyloxyphenoxy]pentyl; 4-[3-pentyloxyphenoxy]butyl; 4-[3-butyloxyphenoxy]butyl; 4-[5-methyl-3-pentyloxyphenoxy]butyl; 4-[5-methoxy-3-pentyloxyphenoxy]butyl; 4-[2,4-dichloro-5-pentyloxyphenoxy]butyl; 4-[2-methyl-3-pentyloxyphenoxy]butyl; 4-[3-cyano-5-pentyloxyphenoxy]butyl; 4-[4-butyloxyphenoxy]butyl; 4-[7-propoxy-2-naphthalenyloxy]butyl; 4-[5-butoxy-1-naphthalenyloxy]butyl; 4-[4-phenoxyphenoxy]butyl; 4-[3-phenoxyphenoxy]butyl; 4-[(3-(4-trifluoromethoxy)phenoxy)phenoxy]butyl; 4-[(4-(4-trifluoromethoxy)phenoxy)phenoxylbutyl; 4-[4-(1-naphthoxy)phenoxy]butyl; 4-[3-(2-naphthoxy)phenoxy]butyl; N-methyl-N-heptyl-hexanamid-6-yl; N-methyl-N-hexyl-heptanamid-7-yl; N-methyl-N-[2-(4-chlorophenoxy)ethyl]hexanamid-6-yl; N-methyl-N-hexanoyl-7-aminoheptyl; N-methyl-N-heptanoyl-6-aminohexyl; N-methyl-N-heptanoyl-5-aminopentyl; N-methyl-N-[2-(4-chlorophenoxy) acetyl]-6-aminohexyl; 5-[3-pentyloxyphenyl]pentyl; 5-[3-hexylphenyl]pentyl; 5-[4-(4-chlorophenoxy)-2-butyloxy]pentyl; 8-(4-aminophenoxy)octyl; 4-[(3-(4-trifluoromethoxy)phenoxy)phenoxy]butyl; or 4-[(4-(4-trifluoromethoxy)phenoxy)phenoxy]butyl;

in free acid form or in salt form.

**5.** (R)-3-Carboxy-N,N,N-trimethyl-2-{[hydroxy(tetradecyloxy)phosphinyl]-oxy}-1-propanaminium hydroxide, inner salt; in free acid or in salt form.

**6.** (R)-3-carboxy-N,N,N-trimethyl-2-{[hydroxy[4-(3-hexyloxyphenoxy)-butyloxy]phosphinyl]oxy}-1-propanaminium hydroxide, inner salt; in free acid or in salt form.

**7.** A process for the preparation of a compound of formula (I) as defined in claim 1 which comprises

a) appropriately reacting a compound of formula (II)

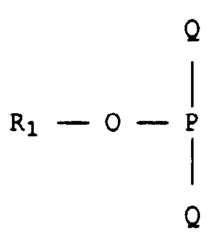

(II)

where $R_1$ is as defined in claim 1 and Q is chlorine, bromine or iodine, with a compound of formula (III)

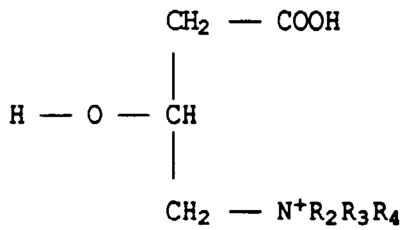

(III)

where $R_2$, $R_3$ and $R_4$ are as defined in claim 1, or a hydrolysable ester thereof, and oxidizing or thiolating the product, and hydrolysing or thiolysing the product; or
b) appropriately reacting a compound of formula ($II^i$)

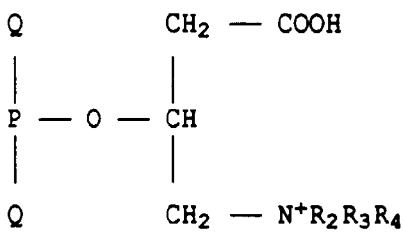

($II^i$)

where

Q is as defined in this claim and
$R_1$, $R_2$ and $R_3$ are as defined in claim 1,

with a compound of formula (IV)

$$R_1 - OH \qquad (IV)$$

where $R_1$ is as defined in claim 1, and oxidizing or thiolating the product, and hydrolysing or thiolysing the product; or
c) for the preparation of the compounds of formula I where a phenyl or naphthyl ring in substituent $R_7$ is mono-, di- or trisubstitued with an $NH_2$ group, reducing a corresponding compound mono-, di- or trisubstituted with an $NO_2$ group;

and recovering the resultant compound in free acid form or in salt, physiologically hydrolysable ester or pro-drug form.

**8.** A pharmaceutical composition comprising a compound of formula (I) as defined in claim 1 in free form or in pharmaceutically acceptable salt or pharmaceutically acceptable and physiologically hydrolysable ester or pro-drug form together with at least one pharmaceutically acceptable carrier or diluent.

**9.** A compound according to claim 1 for use as a pharmaceutical.

**10.** A compound according to claim 9 for use against diabetes.

**Patentansprüche**

**1.** Verbindung der Formel (I):

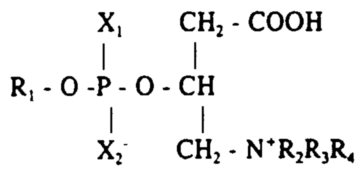

(I)

bei der

$X_1$ und $X_2$ unabhängig O oder S sind;

$R_1$ $R_5$-Y-$R_6$- oder $R_7$-Z-$R_8$- ist, wobei

- Y -O-, -S-, $-CH_2-$, -CH=CH-, -C≡C-, $-N(R_{10})CO-$ oder $-CON(R_{10})-$ ist,
- Z -O-, -S- oder $-CH_2-$ ist,
- $R_5$ gerad- oder verzweigtkettiges $C_{1-17}$-Alkyl oder gerad- oder verzweigtkettiges ω-Trifluor-$C_{1-8}$-Alkyl ist.
- $R_6$ geradkettiges $C_{2-18}$-Alkylen ist, und die Gesamtzahl an Kohlenstoffatomen in $R_5$-Y-$R_6$- 7 bis 19 beträgt;
- $R_7$ nicht-substituiertes Phenyl. Phenoxyphenyl, Biphenyl, Naphthyl oder Naphthoxyphenyl ist, oder Phenyl, Phenoxyphenyl, Biphenyl, Naphthyl oder Naphthoxyphenyl ist, die monosubstituien oder unabhängig di- oder unabhängig tri-substituiert sind mit Halogen, $NO_2$, $NH_2$, CN, $C_{1-8}$-Alkyl, $C_{1-8}$-Alkoxy, Trifluormethyl, Trifluormethoxy oder Acetyl,
- $R_8$ geradkettiges $C_{3-15}$-Alkylen, $-(CH_2)_m-N(R_{10})CO-(CH_2)_n-$, $-(CH_2)_m-CON(R_{10})-(CH_2)_n-$ oder $-CH_2R_{11}OR_{12}-$ ist, wobei m und n unabhängig 1 bis 7 sind,
- $R_{10}$ Wasserstoff, Methyl oder Ethyl ist,
- $R_{11}$ gerad- oder verzweigtkettiges $C_{1-7}$-Alkylen ist;
- $R_{12}$ geradkettiges $C_{2-7}$-Alkylen ist, und die Gesamtzahl an Kohlenstoffatomen in den Arylsubstituenten in $R_7$ und die Gesamtzahl der Kohlenstoffatome in $R_8$ 3 bis 15 beträgt, wobei $R_{10}$ nicht einbezogen wird, und

$R_2$, $R_3$ und $R_4$ jeweils unabhängig gerad- oder verzweigtkettiges $C_{1-4}$-Alkyl ist,

in Form der freien Säure oder in Form eines Salzes, physiologisch hydrolisierbaren Esters oder Pro-Wirkstoffes.

2. Verbindung nach Anspruch 1 der Formel (Is)

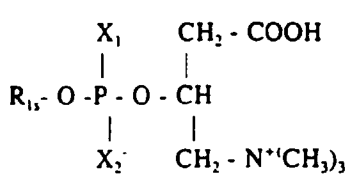

(Is)

bei der

$X_1$ und $X_2$ wie in Anspruch 1 definiert sind, und

$R_{1s}$ $R_5$-$Y_s$-$R_6$- oder $R_{7s}$-Z-$R_{8s}$, ist, wobei

- $Y_s$ -O-, $-CH_2-$, -CH=CH-, -C≡C-, $-N(CH_3)CO-$, $-N(CH_2CH_3)CO-$ oder $-CON(CH_3)-$ ist,
- Z, $R_5$ und $R_6$ wie inAnspruch 1 definiert sind, und die Gesamtzahl an Kohlenstoffatomen in $R_5$-$Y_s$-$R_6$- 7 bis 19 beträgt,
- $R_{7s}$ nicht-substituiertes Phenyl, Phenoxyphenyl, Naphthyl oder Naphthoxyphenyl ist, oder Phenyl, Phenoxyphenyl, Naphthyl oder Naphthoxyphenyl, die mono- oder unabhängig di- oder unabhängig trisubstituiert sind mit Fluor, Chlor, $NO_2$, $NH_2$, CN, $C_{1-6}$-Alkyl, $C_{1-6}$-Alkoxy, Trifluormethyl, Trifluormethoxy oder Acetyl,

$R_{8s}$ geradkettiges $C_{3-12}$-Alkylene, $-(CH_2)_m-N(CH_3)CO-(CH_2)_n-$, $-(CH_2)_m-CON(CH_3)-(CH_2)_n-$ oder $-CH_2R_{11s}OR_{12s}$ ist, wobei

m und n wie in Anspruch 1 definiert sind,
$R_{11s}$ gerad- oder verzweigtkettiges $C_{1-4}$-Alkylen ist,
$R_{12s}$ geradkettiges $C_{2-5}$-Alkylen ist, und

die Gesamtzahl an Kohlenstoffatomen in den Arylsubstituenten in $R_{7s}$ und die Gesamtzahl an Kohlenstoffatomen in $R_{8s}$ 3 bis 15 ist, wobei die an das Stickstoffatom gebundene Methyl gruppe nicht eingerechnet wird,

in Form der freien Säure oder in Form eines Salzes, oder eines Allyl-, Pivaloyloxymethyl- oder N,N-Diethylcarboxamidylmethylcarbonsäure-Esters oder Allylphosphatidorthoesters.

**3.** Verbindung nach Anspruch 1 der Formel (Ip)

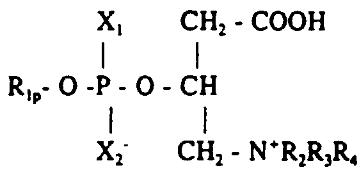

(Ip)

bei der

$X_1$, $X_2$, $R_2$, $R_3$ und $R_4$ wie in Anspruch 1 definiert sind und
$R_{1p}$ $R_{5p}-Y_p-R_{6p}-$ oder $R_{7p}-Z_p-R_{8p}$ ist, wobei

$Y_p$ -O-, -S-, $-CH_2-$, -CH=CH- oder -C≡C- ist,
$Z_p$ -O- oder -S- ist,
$R_{5p}$ gerad- oder verzweigtkettiges $C_{1-17}$-Alkyl ist,
$R_{6p}$ geradkettiges $C_{2-18}$-Alkylen ist, und die Gesamtzahl an Kohlenstoffatomen in $R_{5p}-Y_p-R_{6p}-$ 7 bis 19 beträgt,
$R_{7p}$ nicht-substituiertes Phenyl, Biphenyl oder Naphthyl ist, oder Phenyl oder Naphthyl, die mono- oder unabhängig di- oder unabhängig trisubstituiert sind mit Halogen, $NO_2$, $C_{1-8}$-Alkyl, $C_{1-8}$-Alkoxy, Trifluormethyl, Trifluormethoxy oder Acetyl,
$R_{8p}$ geradkettiges $C_{3-15}$-Alkylen ist, und die Gesamtzahl an Kohlenstoffatomen in den Substituenten $R_{7p}$ und in $R_{8p}$ 3 bis 15 beträgt,

in Form der freien Säure oder in Form eines pharmazeutisch verträglichen Salzes, physiologisch hydrolisierbaren Esters oder Pro-Wirkstoffes.

**4.** Verbindung der Formel (I) nach Anspruch 1, bei der

$R_2$, $R_3$ und $R_4$ Methyl sind, und
$X_1$, $X_2$, $R_1$ bzw. die isomere Form jeweils sind:

S, O, Tetradecyl und R(AB), oder
S, O, Tetradecyl und R(A), oder
S, O, Tetradecyl und R(B), oder
S, S, Tetradecyl und R, oder
S, O, 4-[3-Pentyloxyphenoxy]butyl und R(A), oder
S, O, 4-[3-Pentyloxyphenoxy]butyl und R(B), oder
S, O, 4-[5-Methyl-3-pentyloxyphenoxy]butyl und R(A), oder
S, O, 4-[5-Methyl-3-pentyloxyphenoxy]butyl und R(B), oder
S, O, 8-[4-Trifluormethoxyphenoxy]octyl und R(A), oder

S, O, 8-[4-Trifluormethoxyphenoxy]octyl und R(B), oder
S, O, 4-[3-Hexyloxyphenoxy]butyl und R(A), oder
S, O, 4-[3-Hexyloxyphenoxy]butyl und R(B) oder
S, S, 4-[3-Pentyloxyphenoxy]butyl und R,

oder $X_1$ und $X_2$ O sind und $R_1$ bzw. die isomere Form sind:
Tetradecyl und S,
oder $X_1$ und $X_2$ O sind, die Isomere Form R ist und $R_1$ ist:

Tetradecyl (in der N,N-Diethylcarboxamidylmethylcarbonsäureester-Pro-Wirkstoffsform),
Tetradecyl (in der Pivaloyloxymethylcarbonsäureester-Pro-Wirkstoffsform),
Tetradecyl (in der Allylphosphatidylorthoester-Pro-Wirkstoffsform)
Tetradecyl (in der Allylcarbonsäureesterform),

N-Ethyl-N-heptyl-hexanamid-6-yl, 8-(2-Aminophenoxy)octyl, Tridecyl, Pentadecyl, 7-(Z)-Tetradecenyl, 11-(Z)-Tetradecenyl, 7-Tetradecynyl, 4-Nonyloxybutyl, 12-Methyloxydodecyl, 6-Heptyloxyhexyl, 8-Pentyloxyoctyl, 10-Propyloxydecyl. 2-Undecyloxyethyl, 9-(3,3-Dimethylbutyloxy)nonyl, 5-Octyloxypentyl, 5-(3-Decyloxy)pentyl, 9-(4,4,4-Trifluorbutyloxy)nonyl, 6-(2-Nonyloxy)pentyl, 6-(2-Octyloxy)hexyl, 4-(2-Decyloxy)butyl, 8-[2-(2-Methyl)hexyloxy]-octyl, 9-[2-(2-Methyl)pentyloxy]nonyl, 7-[2-(2-Methyl)heptyloxy]heptyl, 5-[2-(2-Methyl)-nonyloxy]pentyl, 8-(3-Methylpentyloxy)octyl, 8-[4-(Trifluormethoxy)phenoxy]octyl, 7-Phenoxyheptyl, 7-(4-Chlorphenoxy)heptyl, 6-(4-Chlorphenoxy)hexyl, 8-(4-chlorphenoxy)octyl, 9-(4-Chlor-phenoxy)nonyl, 8-(2-Chlorphenoxy)octyl, 8-(3-Chlorphenoxy)octyl, 7-(1-Naphthyloxy)heptyl, 7-(2-Naphthyloxy)heptyl, 7-(3,5-Di-Trifluormethylphenoxy)heptyl, 8-(4-tert.-Butylphenoxy)octyl, 8-(4-Phenylphenoxy)octyl, 8-(4-Acetyl-3-methylphenoxy)octyl, 4-[3-(2-Naphthoxy) phenoxy]butyl, 8-[(4-Chlorphenyl)thio]octyl, 10-(4-Chlorphenoxy)decyl, 8-(3,5-Dimethoxyphenoxy)octyl, 8-(2,3,4-Trichlorphenoxy)octyl, 8-(2,5-Dinitrophenoxy)octyl, 8-(2,3-Dimethylphenoxy)octyl, 8-(3,4-Dimethylphenoxy)octyl, 8-(3-Fluor-4-nitrophenoxy)octyl, 8-(2,4-Dimethylphenoxy)octyl, 8-(4-Nitrophenoxy)octyl, 8-(3-Nitrophenoxy)octyl, 8-(2,4-Dinitrophenoxy)octyl, 8-(2,4-Dichlorphenoxy)octyl, 8-(3-Trifluormethoxyphenoxy)octyl, 8-(2-Trifluormethylphenoxy)octyl, 8-(4-Methoxyphenoxy)octyl, 4-(6-Propoxy-2-naphthoxy)butyl, 8-(2,3-Dichlorphenoxy)octyl, 8-(2,5-Dichlorphenoxy)octyl, 8-(4-Methylphenoxy)octyl, 8-(4-Trifluormethylphenoxy)octyl, 8-(2-Nitrophenoxy)octyl, 9-(4-Trifluormethoxyphenoxy)nonyl, 8-(2,6-Dichlorphenoxy)octyl, 3-[3-Pentyloxyphenoxy]propyl. 3-[3-Hexyloxyphenoxy]propyl, 5-[3-Butyloxyphenoxy]pentyl, 5-[3-Pentyloxyphenoxy]pentyl, 4-[3-Pentyloxyphenoxy]butyl, 4-[3-Butyloxyphenoxy]butyl, 4-[5-Methyl-3-pentyloxyphenoxy]butyl, 4-[5-Methoxy-3-Pentyloxyphenoxy] butyl, 4-[2,4-Dichlor-5-pentyloxyphenoxy]butyl, 4-[2-Methyl-3-pentyloxyphenoxy]butyl, 4-[3-Cyano-5-pentyloxyphenoxy]butyl, 4-[4-Butyloxyphenoxy]butyl, 4-[7-Propoxy-2-naphthalenyloxy]butyl, 4-[5-Butoxy-1-Naphthalenyloxy]butyl, 4-[4-Phenoxyphenoxy]butyl, 4-[3-Phenoxyphenoxy]butyl, 4-[(3-(4-Trifluormethoxy)phenoxy)phenoxy]butyl, 4-[(4-(4-Trifluormethoxy)phenoxy)phenoxy]butyl, 4-[4-(1-Naphthoxy)phenoxy]butyl, 4-[3-(2-Naphthoxy)phenoxy]butyl, N-Methyl-N-heptylhexanamid-6-yl, N-Methyl-N-hexyl-heptanamid-7-yl, N-Methyl-N-[2-(4-chlorphenoxy) ethyl]hexanamid-6-yl, N-Methyl-N-hexanoyl-7-aminoheptyl, N-Methyl-N-heptanoyl-6-aminohexyl, N-Methyl-N-heptanoyl-5-aminopentyl, N-Methyl-N-[2-(4-chlorphenoxy)acetyl]-6-aminohexyl, 5-[3-Pentyloxyphenyl]pentyl, 5-[3-Hexylphenyl]pentyl, 5-[4-(4-Chlorphenoxy)-2-butyloxy]pentyl, 8-(4-Aminophenoxy)octyl, 4-[(3-(4-Trifluormethoxy)phenoxy)phenoxy]butyl, oder 4-[(4-(4-Trifluormethoxy)phenoxy)phenoxy]butyl, in Form freier Säure oder Salzform.

**5.** (R)-3-Carboxy-N,N,N-trimethyl-2-{[hydroxy(tetradecyloxy)phosphinyl]-oxy}-1-propanaminiumhydroxid, inneres Salz, in Form freier Säure oder Salzform.

**6.** (R)-3-Carboxy-N,N,N-trimethyl-2-{[hydroxy[4-(3-hexyloxyphenoxy)-butyloxy]phosphinyl]oxy}-1-propanaminiumhydroxid, inneres Salz, in Form freier Säure oder Salzform.

**7.** Verfahren zur Herstellung einer Verbindung der Formel (I) nach Anspruch 1, welches umfaßt

a) geeignetes Umsetzen einer Verbindung der Formel (II)

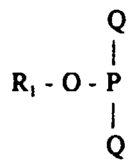

(II)

wobei $R_1$ wie in Anspruch 1 definiert ist und Q Chlor, Brom oder Jod ist, mit einer Verbindung der Formel (III)

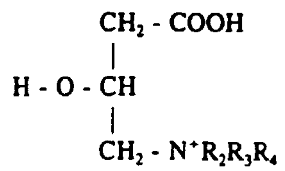

(III)

wobei $R_2$, $R_3$ und $R_4$ wie inAnspruch 1 definiert sind, oder einem hydrolisierbaren Ester davon, und Oxidieren oder Thiolieren des Produktes, und Hydrolisieren oder Thiolisieren des Produktes, oder
b) geeignetes Umsetzen einer Verbindung der Formel (II')

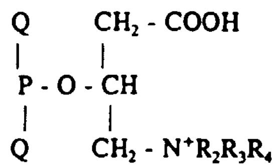

(II')

wobei Q wie in diesem Anspruch definiert ist und $R_2$, $R_3$ und $R_4$ wie in Anspruch 1 definiert sind mit einer Verbindung der Formel (IV)

$$R_1\text{ - OH} \qquad \text{(IV)}$$

wobei $R_1$ wie in Anspruch 1 definiert ist, und Oxidieren oder Thiolieren des Produktes und Hydrolisieren oder Thiolisieren des Produktes, oder
c) zur Herstellung der Verbindungen der Formel (I), bei denen im Substituenten $R_7$ ein Phenyl- oder Naphthylring mit einer $NH_2$-Gruppe mono-, di- oder trisubstituiert ist, Reduzieren einer entsprechenden Verbindung, die mit einer $NO_2$-Gruppe mono-, di- oder trisubstituiert ist,

und Gewinnen der so erhaltenen Verbindung in Form der freien Säure oder in Form eines Salzes, physiologisch hydrolisierbaren Esters oder Pro-Wirkstoffes.

**8.** Pharmazeutische Zusammensetzung, welche eine Verbindung der Formel (I) nach Anspruch 1 in freier Form oder in Form eines pharmazeutisch verträglichen Salzes oder pharmazeutisch verträglichen und physiologisch hydrolysierbaren Esters oder Pro-Wirkstoffes zusammen mit mindestens einem phannazeutisch verträglichen Träger oder Verdünnungsmittel enthält.

**9.** Verbindung nach Anspruch 1 zur Verwendung als Arzneimittel.

**10.** Verbindung nach Anspruch 9 zur Verwendung gegen Diabetes.

**Revendications**

**1.** Un composé de formule (I)

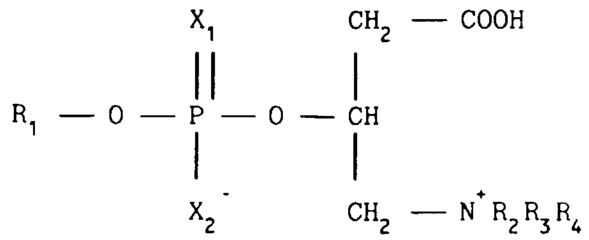

(I)

dans laquelle

$X_1$ et $X_2$ signifient indépendamment O ou S;

$R_1$ signifie $R_5$-Y-$R_6$- ou $R_7$-Z-$R_8$- où

Y signifie -O-, -S-, $-CH_2-$, -CH=CH-, -C≡C-, -N($R_{10}$)CO- ou -CON($R_{10}$)-;

Z signifie -O-, -S- ou $-CH_2-$;

$R_5$ signifie un groupe ($C_{1-17}$)alkyle à chaîne droite ou ramifiée ou un groupe ω-trifluoro-($C_{1-8}$)alkyle à chaîne droite ou ramifiée;

$R_6$ signifie un groupe ($C_{2-18}$)alkylène à chaîne droite; et le nombre total d'atomes de carbone dans $R_5$-Y-$R_6$- est compris entre 7 et 19;

$R_7$ signifie un groupe phényle, phénoxyphényle, biphényle, naphtyle ou naphtoxyphényle non substitués; ou phényle, phénoxyphényle, biphényle, naphtyle ou naphtoxyphényle mono- ou indépendamment di- ou indépendamment trisubstitués par un halogène, $NO_2$, $NH_2$, CN, ($C_{1-8}$)alkyle, ($C_{1-8}$)alcoxy, trifluorométhyle, trifluorométhoxy ou acétyle;

$R_8$ signifie un groupe ($C_{3-15}$)alkylène à chaîne droite, $-(CH_2)_m$-N($R_{10}$)CO-$(CH_2)_n$-, -$(CH_2)_m$-CON($R_{10}$)-$(CH_2)_n$- ou $-CH_2R_{11}OR_{12}$- où m et n signifient indépendamment de 1 à 7;

$R_{10}$ signifie l'hydrogène, un groupe méthyle ou éthyle;

$R_{11}$ signifie un groupe ($C_{1-7}$)alkylène à chaîne droite ou ramifiée;

$R_{12}$ signifie un groupe ($C_{2-7}$)alkylène à chaîne droite; et le nombre total d'atomes de carbone dans les substituants aryle dans $R_7$, et le nombre total d'atomes de carbone dans $R_8$, sans compter la signification de $R_{10}$, est compris entre 3 et 15; et

$R_2$, $R_3$ et $R_4$ signifient chacun indépendamment un groupe ($C_{1-4}$)alkyle à chaîne droite ou ramifiée;

sous forme d'acide libre ou sous forme d'un sel, d'un ester physiologiquement hydrolysable ou sous forme d'un promédicament.

**2.** Un composé selon la revendication 1 de formule (Is)

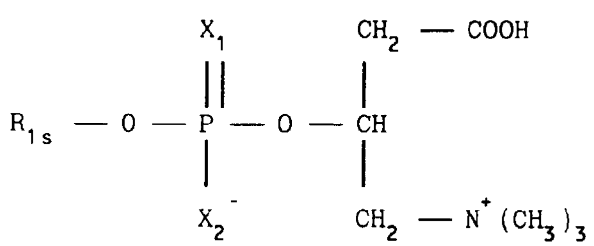

(Is)

dans laquelle

$X_1$ et $X_2$ sont tels que définis à la revendication 1; et

$R_{1s}$ signifie $R_5$-$Y_s$-$R_6$- ou $R_{7s}$-Z-$R_{8s}$- où

$Y_s$ signifie -O-, $-CH_2-$, -CH=CH-, -C≡C-, $-N(CH_3)CO-$, $-N(CH_2CH_3)CO-$ ou bien $-CON(CH_3)-$;

Z, $R_5$ et $R_6$ sont tels que définis à la revendication 1; et le nombre total d'atomes de carbone dans $R_5$-$Y_s$-$R_6$- est compris entre 7 et 19;

$R_{7s}$ signifie un groupe phényle, phénoxyphényle, naphtyle ou naphtoxyphényle non substitués; ou bien phényle, phénoxyphényle, naphtyle ou naphtoxyphényle mono- ou indépendamment di-ou indépendamment trisubstitués par du fluor, du chlore, $NO_2$, $NH_2$, CN, $(C_{1-6})$alkyle, $(C_{1-6})$-alcoxy, trifluorométhyle, trifluorométhoxy ou acétyle;

$R_{8s}$ signifie un groupe $(C_{3-12})$alkylène à chaîne droite, $-(CH_2)_m-N(CH_3)CO-(CH_2)_n-$, $-(CH_2)_m-CON(CH_3)-(CH_2)_n-$ ou bien $-CH_2R_{11s}OR_{12s}$ où m et n sont tels que définis à la revendication 1,

$R_{11s}$ signifie un groupe $(C_{1-4})$alkylène à chaîne droite ou ramifiée;
$R_{12s}$ signifie un groupe $(C_{2-5})$alkylène à chaîne droite; et

le nombre total d'atomes de carbone dans les substituants aryle dans $R_{7s}$ et le nombre total d'atomes de carbone dans $R_{8s}$, sans compter le groupe méthyle fixé à l'atome d'azote, est compris entre 3 et 15;

sous forme d'acide libre ou d'un sel, ou sous forme d'un groupe allyle, d'un carboxylate de pivaloyloxyméthyle ou de N,N-diéthylcarboxamidylméthyle ou d'un orthoester allylphosphatidique.

**3.** Un composé selon la revendication 1 de formule (Ip)

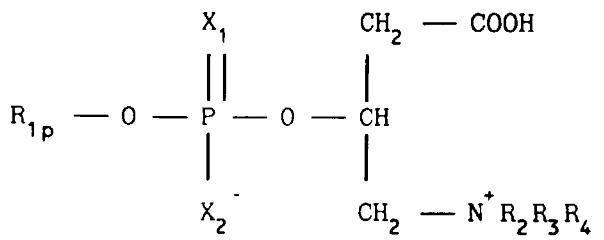

(Ip)

dans laquelle $X_1$, $X_2$, $R_2$, $R_3$ et $R_4$ sont tels que définis à la revendication 1, et

$R_{1p}$ signifie $R_{5p}$-$Y_p$-$R_{6p}$- ou bien $R_{7p}$-$Z_p$-$R_{8p}$- où

$Y_p$ signifie -O-, -S-, $-CH_2-$, -CH=CH- ou -C≡C-;
$Z_p$ signifie -O- ou -S-;
$R_{5p}$ signifie un groupe $(C_{1-17})$alkyle à chaîne droite ou ramifiée;
$R_{6p}$ signifie un groupe $(C_{2-18})$alkylène à chaîne droite; et le nombre total d'atomes de carbone dans $R_{5p}$-$Y_p$-$R_{6p}$- est compris entre 7 et 19;
$R_{7p}$ signifie un groupe phényle, biphényle ou naphtyle non substitués; ou bien un groupe phényle ou naphtyle mono- ou indépendamment di- ou indépendamment trisubstitué par un halogène, $NO_2$, $(C_{1-8})$alkyle, $(C_{1-8})$-alcoxy, trifluorométhyle, trifluorométhoxy ou acétyle;
$R_{8p}$ signifie un groupe $(C_{3-15})$alkylène à chaîne droite; et le nombre total d'atomes de carbone dans les substituants dans $R_{7p}$ et dans $R_{8p}$ est compris entre 3 et 15;

sous forme d'acide libre ou d'un sel pharmaceutiquement acceptable, d'un ester physiologiquement hydrolysable ou d'un promédicament.

**4.** Le composé selon la revendication 1 de formule (I) où $R_2$, $R_3$ et $R_4$ signifient un groupe méthyle, et

soit $X_1$, $X_2$, $R_1$ et la forme isomère signifient respectivement:

S, O, tétradécyle et R(AB); ou bien

S, O, tétradécyle et R(A); ou bien
S, O, tétradécyle et R(B); ou bien
S, S, tétradécyle et R; ou bien
S, O, 4-[3-pentyloxyphénoxy]butyle et R(A); ou bien
S, O, 4-[3-pentyloxyphénoxy]butyle et R(B); ou bien
S, O, 4-[5-méthyl-3-pentyloxyphénoxy]butyle et R(A); ou bien
S, O, 4-[5-méthyl-3-pentyloxyphénoxy]butyle et R(B); ou bien
S, O, 8-[4-trifluorométhoxyphénoxy]octyle et R(A); ou bien
S, O, 8-[4-trifluorométhoxyphénoxy]octyle et R(B); ou bien
S, O, 4-[3-hexyloxyphénoxy]butyle et R(A); ou bien
S, O, 4-[3-hexyloxyphénoxy]butyle et R(B); ou bien
S, S, 4-[3-pentyloxyphénoxy]butyle et R;

ou bien $X_1$ et $X_2$ signifient O et $R_1$ et la forme isomère signifient respectivement: tétradécyle et S;

ou bien $X_1$ et $X_2$ signifient O, la forme isomère signifie R, et $R_1$ signifie:

tétradécyle (sous forme d'un promédicament du carboxylate de N,N-diéthylcarboxamidylméthyle); tétradécyle (sous forme d'un promédicament du carboxylate de pivaloyloxyméthyle); tétradécyle (sous forme d'un promédicament de l'orthoester allylphosphatidique); tétradécyle (sous forme de carboxylate d'allyle); N-éthyl-N-heptyl-hexanamid-6-yle; 8-(2-aminophénoxy)octyle; tridécyle; pentadécyle; 7-(Z)-tétradécényle; 11-(Z)-tétradécényle; 7-tétradécynyle; 4-nonyloxybutyle; 12-méthyloxydodécyle; 6-heptyloxyhexyle; 8-pentyloxyoctyle; 10-propyloxydécyle; 2-undécyloxyéthyle; 9-(3,3-diméthylbutyloxy)nonyle; 5-octyloxypentyle; 5-(3-décyloxy)pentyle; 9-(4,4,4-trifluorobutyloxy)nonyle; 6-(2-nonyloxy)pentyle; 6-(2-octyloxy)hexyle; 4-(2-décyloxy)butyle; 8-[2-(2-méthyl)hexyloxy]octyle; 9-[2-(2-méthyl)pentyloxy]nonyle; 7-[2-(2-méthyl)heptyloxy]heptyle;

5-[2-(2-méthyl)nonyloxy]pentyle; 8-(3-méthylpentyloxy)octyle;
8-[4-trifluorométhoxy)phénoxy]octyle; 7-phénoxyheptyle;
7-(4-chlorophénoxy)heptyle; 6-(4-chlorophénoxy)hexyle;
8-(4-chlorophénoxy)octyle; 9-(4-chlorophénoxy)nonyle;
8-(2-chlorophénoxy)octyle; 8-(3-chlorophénoxy)octyle;
7-(1-naphtyloxy)heptyle; 7-(2-naphtyloxy)heptyle;
7-(3,5-di-trifluorométhylphénoxy)heptyle; 8-(4-tert.-butylphénoxy)octyle;
8-(4-phénylphénoxy)octyle; 8-(4-acétyl-3-méthylphénoxy)octyle;
4-[3-(2-naphtoxy)phénoxy]butyle; 8-[(4-chlorophényl)thio]octyle;
10-(4-chlorophénoxy)décyle; 8-(3,5-diméthoxyphénoxy)octyle;
8-(2,3,4-trichlorophénoxy)octyle; 8-(2,5-dinitrophénoxy)octyle;
8-(2,3-diméthylphénoxy)octyle; 8-(3,4-diméthylphénoxy)octyle;
8-(3-fluoro-4-nitrophénoxy)octyle; 8-(2,4-diméthylphénoxy)octyle;
8-(4-nitrophénoxy)octyle; 8-(3-nitrophénoxy)octyle;
8-(2,4-dinitrophénoxy)octyle; 8-(2,4-dichlorophénoxy)octyle;
8-(3-trifluorométhoxyphénoxy)octyle; 8-(2-trifluorométhylphénoxy)octyle;
8-(4-méthoxyphénoxy)octyle; 4-(6-propoxy-2-naphtoxy)butyle;
8-(2,3-dichlorophénoxy)octyle; 8-(2,5-dichlorophénoxy)octyle;
8-(4-méthylphénoxy)octyle; 8-(4-trifluorométhylphénoxy)octyle;
8-(2-nitrophénoxy)octyle; 9-(4-trifluorométhoxyphénoxy)nonyle;
8-(2,6-dichlorophénoxy)octyle; 3-[3-pentyloxyphénoxy]propyle;
3-[3-hexyloxyphénoxy]propyle; 5-[3-butyloxyphénoxy]pentyle;
5-[3-pentyloxyphénoxy]pentyle; 4-[3-pentyloxyphénoxy]butyle;
4-[3-butyloxyphénoxy]butyle; 4-[5-méthyl-3-pentyloxyphénoxy]butyle;
4-[5-méthoxy-3-pentyloxyphénoxy]butyle;
4-[2,4-dichloro-5-pentyloxyphénoxy]butyle;
4-[2-méthyl-3-pentyloxyphénoxy]butyle; 4-[3-cyano-5-pentyloxyphénoxy]butyle;
4-[4-butyloxyphénoxy]butyle; 4-[7-propoxy-2-naphtalenyloxy]butyle;
4-[5-butoxy-1-naphtalenyloxy]butyle; 4-[4-phénoxyphénoxy]butyle;
4-[3-phénoxyphénoxy]butyle;
4-[(3-(4-trifluorométhoxy)phénoxy)phénoxy]butyle;
4-[(4-(4-trifluorométhoxy)phénoxy)phénoxy]butyle;

4-[4-(1-naphtoxy)phénoxy]butyle; 4-[3-(2-naphtoxy)phénoxy]butyle; N-méthyl-N-heptyl-hexanamid-6-yle; N-méthyl-N-hexyl-heptanamid-7-yle; N-méthyl-N-[2-(4-chlorophénoxy)éthyl]hexanamid-6-yle; N-méthyl-N-hexanoyl-7-aminoheptyle; N-méthyl-N-heptanoyl-6-aminohexyle; N-méthyl-N-heptanoyl-5-aminopentyle; N-méthyl-N-[2-(4-chlorophénoxy)acétyl]-6-aminohexyle; 5-[3-pentyloxyphényl]pentyle; 5-[3-hexylphényl]pentyle; 5-[4-(4-chlorophénoxy)-2-butyloxy]pentyle; 8-(4-aminophénoxy)octyle; 4-[(3-(4-trifluorométhoxy)phénoxy)phénoxy]butyle; ou bien 4-[(4-(4-trifluorométhoxy)phénoxy)phénoxy]butyle;

sous forme d'acide libre ou sous forme d'un sel.

5. Hydroxyde de (R)-3-carboxy-N,N,N-triméthyl-2-{[hydroxy(tétradécyloxy)phosphinyl]-oxy}-1-propanaminium, sel interne; sous forme d'acide libre ou sous forme d'un sel.

6. Hydroxyde de (R)-3-carboxy-N,N,N-triméthyl-2-{[hydroxy([4-(3-hexyloxyphénoxy)-butyloxy]phosphinyl]-oxy}-1-propanaminium, sel interne; sous forme d'acide libre ou sous forme d'un sel.

7. Un procédé de préparation d'un composé de formule (I) tel que défini à la revendication 1, selon lequel

a) on fait réagir de façon appropriée un composé de formule (II)

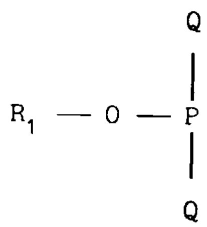

(II)

où $R_1$ est tel que défini à la revendication 1 et Q signifie le chlore, le brome ou l'iode, avec un composé de formule (III)

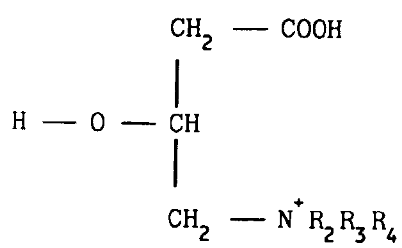

(III)

où $R_2$, $R_3$ et $R_4$ sont tels que définis à la revendication 1, ou un ester hydrolysable de ce composé, et on soumet le produit à une oxydation ou à une thiolation et on hydrolyse ou on thiolyse le produit; ou bien
b) on fait réagir de façon appropriée un composé de formule ($II^i$)

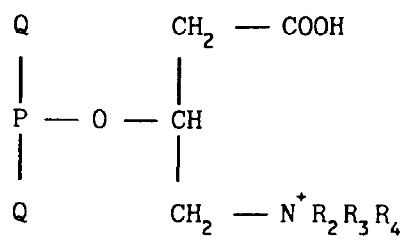

($II^i$)

où Q est tel que défini dans cette revendication, et

$R_1$, $R_2$ et $R_3$ sont tels que définis à la revendication 1,

avec un composé de formule (IV)

$$R_1\text{-OH} \qquad (IV)$$

où $R_1$ est tel que défini à la revendication 1, et on soumet le produit à une oxydation ou à une thiolation et on hydrolyse ou on thiolyse le produit; ou bien

c) pour la préparation des composés de formule I où un cycle phényle ou naphtyle dans le substituant $R_7$ est mono-, di- ou trisubstitué par un groupe $NH_2$, on réduit un composé correspondant mono-, di- ou trisubstitué par un groupe $NO_2$;

et on récupère le composé résultant sous forme d'acide libre ou sous forme d'un sel, d'un ester physiologiquement hydrolysable ou sous forme d'un promédicament.

**8.** Une composition pharmaceutique comprenant un composé de formule (I) tel que défini à la revendication 1, sous forme libre ou sous forme d'un sel pharmaceutiquement acceptable ou d'un ester pharmaceutiquement acceptable et physiologiquement hydrolysable ou sous forme d'un promédicament, ensemble avec au moins un véhicule ou diluant pharmaceutiquement acceptable.

**9.** Un composé selon la revendication 1, pour une utilisation comme médicament.

**10.** Un composé selon la revendication 9, pour une utilisation contre le diabète.